(19) Europäisches Patentamt
European Patent Office
Office européen des brevets

(11) EP 1 359 219 A1

(12) # EUROPEAN PATENT APPLICATION
published in accordance with Art. 158(3) EPC

(43) Date of publication:
**05.11.2003 Bulletin 2003/45**

(21) Application number: **02711315.8**

(22) Date of filing: **06.02.2002**

(51) Int Cl.⁷: **C12N 15/11**, C12Q 1/68

(86) International application number:
**PCT/JP02/00941**

(87) International publication number:
**WO 02/062993 (15.08.2002 Gazette 2002/33)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **06.02.2001 JP 2001030153**

(71) Applicant: **TAKARA BIO INC.**
**Otsu-shi, Shiga 520-2193 (JP)**

(72) Inventors:
- **MINENO, Junichi**
  **Uji-shi, Kyoto 611-0002 (JP)**
- **TAKEDA, Osamu**
  **Hikone-shi, Shiga 521-1124 (JP)**
- **ROKUSHIMA, Masatomo**
  **Matsudo-shi, Chiba 270-0023 (JP)**
- **UEMORI, Takashi**
  **Otsu-shi, Shiga 520-2141 (JP)**
- **HOKAZONO, Shigekazu**
  **Otsu-shi, Shiga 520-2134 (JP)**
- **MUKAI, Hiroyuki**
  **Moriyama-shi, Shiga 524-0102 (JP)**
- **YAMASHITA, Shusaku**
  **Otshi-shi, Shiga 520-2134 (JP)**
- **SAGAWA, Hiroaki**
  **Kusatsu-shi, Shiga 520-0025 (JP)**
- **ASADA, Kiyozo**
  **Koka-gun, Shiga 520-3333 (JP)**
- **KATO, Ikunoshin**
  **Uji-shi, Kyoto 611-0028 (JP)**

(74) Representative: **Grund, Martin, Dr. et al**
**Dr. Volker Vossius,**
**Patentanwaltskanzlei,**
**Geibelstrasse 6**
**81679 München (DE)**

(54) **AMPLIFIED NUCLEIC ACIDS AND IMMOBILIZED PRODUCTS THEREOF**

(57) A nucleic acid, which is provided in a large amount through a nucleic acid amplification reaction with the use of chimeric oligonucleotide primers, is constructed in a state of containing a modified deoxyribonucleotide for immobilizing the nucleic acid to a solid phase and then immobilized to a solid phase at a high efficiency, thereby giving an immobilized nucleic acid product with excellent qualities.

EP 1 359 219 A1

## Description

Technical Field

The present invention relates to a highly sensitive DNA chip which is useful in a field of gene analysis.

Background Art

A substrate onto which a nucleic acid is immobilized (e.g., a DNA chip) has been developed as a tool for rapidly promoting a gene function analysis project. It has been widely used for analyzing expression of all yeast genes as well as genes in cultured cells and tissues. Known methods for preparing DNA chips (or DNA arrays) include, for example, a method in which a single-stranded DNA is synthesized in a predefined region on a chip solid phase, and a method in which a single-stranded or double-stranded DNA which has been prepared beforehand is spotted in a predefined region on a chip solid phase. DNA chips prepared according to the former method are exemplified by high-density oligonucleotide arrays as described in United States Patent Nos. 5,445,934, 5,744,305 and 5,700,637. DNA chips prepared according to the latter method are exemplified by spotting arrays as described in United States Patent No. 5,807,522 and WO 98/18961. A DNA chip can be prepared by spotting a DNA in a predefined region on a chip solid phase using this method. For example, a DNA fragment is physically spotted using a pin tip onto a solid phase substrate such as a slide glass which has been subjected to special processing such as poly-L-lysine-coating or silanization. In this case, the DNA fragment electrostatically binds to the solid phase substrate. Accordingly, the binding efficiency may vary depending on the conditions of coating on the solid phase substrate. On the other hand, a large-scale instrument for solid phase synthesis at high density is required for the preparation of the above-mentioned high-density oligonucleotide array. Furthermore, it is difficult to synthesize a long nucleotide chain according to this method because of its synthesis yield.

In both preparation methods, it is necessary to synthesize a nucleic acid to be immcbilized in large quantities. Most of nucleic acid amplification methods with the exception of synthesis of a short-chain DNA such as an oligonucleotide are carried out by enzymatic means utilizing a DNA polymerase. An example of the methods is the polymerase chain reaction (PCR) method as described in United States Patent Nos. 4,683,195, 4,683,202 and 4,800,159 in detail. Another example is the reverse transcription-PCR (RT-PCR) method, which is a combination of the PCR and a reverse transcriptase reaction, as described in Trends in Biotechnology, 10:146-152 (1992). The development of the above-mentioned methods has enabled amplification of a region of interest from a DNA or an RNA.

The above-mentioned DNA synthesis methods are conducted, for example, using a reaction that consists of three steps. The three steps are a step of dissociating (denaturing) a double-stranded DNA into single-stranded DNAs, a step of annealing a primer to the single-stranded DNA and a step of synthesizing (extending) a complementary strand from the primer in order to amplify a region of a DNA of interest. Alternatively, they are conducted using a reaction designated as "the shuttle PCR" ("PCR hou saizensen" (Recent advances in PCR methodology), Tanpakushitsu Kakusan Kouso, Bessatsu, (Protein, Nucleic Acid and Enzyme, Supplement), 41(5):425-428 (1996)) in which two of the three steps, that is, the step of annealing the primer and the step of extending are carried out at the same temperature.

Alternatively, the ligase chain reaction (LCR) method as described in EP 320,308 published on June 14, 1989 or the transcription-based amplification system (TAS) method as described in PCR Protocols, Academic Press Inc., 1990, pp. 245-252 may be used. The four methods as mentioned above require repeating reactions at a high temperature and a low temperature several times in order to regenerate a single-stranded target molecule for the next amplification cycle. The reaction system should be conducted using discontinuous phases or cycles because the reaction is restricted by temperatures as described above. Thus, the methods require the use of an expensive thermal cycler that can strictly adjust a wide range of temperatures over time. Furthermore, the reaction requires time for adjusting the temperature to the two or three predefined ones. The loss of time increases in proportion to the cycle number.

Nucleic acid amplification methods that can be carried out isothermally have been developed in order to solve the problems. Examples thereof include the strand displacement amplification (SDA) method as described in Japanese Patent Nos. 2,076,096 and 2,087,487, the self-sustained sequence replication (3SR) method, the nucleic acid sequence based amplification (NASBA) method as described in Japanese Patent No. 2,650,159, the transcription-mediated amplification (TMA) method, the Qβ replicase method as described in Japanese Patent No. 2,710,159 and the various modified SDA methods as described in United States Patent No. 5,824,517, WO 99/09211, WO 95/25180 and WO 99/49081. A method of isothermal enzymatic synthesis of an oligonucleotide is described in United States Patent No. 5,916,777. Extension from a primer and/or annealing of a primer to a single-stranded extension product (or to an original target sequence) followed by extension from the primer take place in parallel in a reaction mixture incubated at a constant temperature in the reaction of these methods of isothermal nucleic acid amplification or synthesis of an oligonucleotide.

Among the isothermal nucleic acid amplification methods, the SDA method is an example of systems in which a DNA is finally amplified. The SDA method is a method for amplifying a target nucleic acid sequence (and a complementary strand thereof) in a sample by displacement of double strands using a DNA polymerase and a restriction endonuclease. The method requires four primers used for the amplification, two of which should be designed to contain a recognition site for the restriction endonuclease. The method requires the use of a modified deoxyribonucleotide triphosphate as a substrate for DNA synthesis in large quantities. An example of the modified deoxyribonucleotide triphosphates is an (α-S) deoxyribonucleotide triphosphate in which the oxygen atom of the phosphate group at the α-position is replaced by a sulfur atom (S). The problem of running cost associated with the use of the modified deoxyribonucleotide triphosphate becomes serious if the reaction is routinely conducted, for example, for genetic test. Furthermore, the incorporation of the modified nucleotide such as the (α-S) deoxyribonucleotide into the amplified DNA fragment in the method may abolish the cleavability of the amplified DNA fragment with a restriction enzyme, for example, when it is subjected to a restriction enzyme fragment length polymorphism (RFLP) analysis.

The modified SDA method as described in United States Patent No. 5,824,517 is a DNA amplification method that uses a chimeric primer that is composed of an RNA and a DNA and has as an essential element a structure in which DNA is positioned at least at the 3' terminus. The modified SDA method as described in WO 99/09211 requires the use of a restriction enzyme that generates a 3'-protruding end. The modified. SDA method as described in WO 95/25180 requires the use of at least two pairs of primers. The modified SDA method as described in WO 99/49081 requires the use of at least two pairs of primers and at least one modified deoxyribonucleotide triphosphate. On the other hand, the method for synthesizing an oligonucleotide as described in United States Patent No. 5,916,777 comprises synthesizing a DNA using a primer having a ribonucleotide at the 3' terminus, completing a reaction using the primer, introducing a nick between the primer and an extended strand in an primer-extended strand with an endonuclease to separate them, digesting a template and recovering the primer to reuse it. It is required to isolate the primer from the reaction system and then anneal it to the template again in order to reuse the primer in the method. Additionally, the LAMP method as described in WO 00/28082 requires four primers for amplification and the products amplified using the method are DNAs having varying size in which the target regions for the amplification are repeated.

As described above, it was difficult to produce and supply a nucleic acid to be used for a substrate having an immobilized nucleic acid in large quantities according to the conventional nucleic acid amplification methods. The ICAN method as described in WO 00/56877 may be used to produce a nucleic acid in large quantities and to produce a material having an immobilized nucleic acid at a low cost. However, the method has problems as follows. A ribonucleotide may be contained in a fragment amplified according to this method. If the amplified fragment is immobilized onto a solid phase, the immobilized nucleic acid may be degraded and released upon certain aftertreatment.

Objects of Invention

The main object of the present invention is to prepare a nucleic acid, which is supplied in large quantities by a nucleic acid amplification reaction utilizing a chimeric oligonucleotide primer, such that the nucleic acid contains a deoxyribonucleotide modified for immobilization onto a solid phase, and to obtain a material having an immobilized nucleic acid of a high quality by immobilizing the nucleic acid onto a solid phase with high efficiency.

Summary of Invention

As a result of intensive studies, the present inventors have constructed a method for producing a nucleic acid, which is used for preparing a material having an immobilized nucleic acid, utilizing the isothermal and chimeric primer-initiated amplification of nucleic acids (ICAN} method. The ICAN method is a nucleic acid amplification method in which a region of interest in a DNA is amplified in the presence of a chimeric oligonucleotide primer in which a ribonucleotide is positioned at the 3' terminus or on the 3'-terminal side, an endoribonuclease and a DNA polymerase.

Furthermore, the present inventors could obtain an ICAN reaction product containing a modified deoxyribonucleotide by carrying out the ICAN method using a primer or a deoxyribonucleotide triphosphate modified for immobilizing an amplified nucleic acid onto a solid phase. The present inventors have constructed a method for producing a material having an immobilized nucleic acid by immobilizing the thus obtained ICAN reaction product containing a modified deoxyribonucleotide onto a solid phase. Thus, the present invention has been completed.

The first aspect of the present invention relates to an amplified nucleic acid containing a deoxyribonucleotide modified for immobilization onto a solid phase, which is obtained by a nucleic acid amplification method comprising:

(a) preparing a reaction mixture by mixing a nucleic acid as a template, deoxyribonucleotide triphosphates, a DNA polymerase having a strand displacement activity, at least one primer and an RNase H, wherein the primer is a chimeric oligonucleotide primer that is substantially complementary to the nucleotide sequence of the nucleic acid as the template and contains deoxyribonucleotides and a ribonucleotide, the ribonucleotide being positioned at the 3' terminus or on the 3'-terminal side of the primer, wherein a part of the deoxyribonucleotide triphosphates is a deoxyribonucleotide triphosphate modified for immobilization onto a solid phase and/or a part of the deoxyribonucleotides is a deoxyribonucleotide modified for immobilization onto a solid phase; and
(b) incubating the reaction mixture for a sufficient time to generate a reaction product.

According to the first aspect, the deoxyribonucleotide modified for immobilization onto a solid phase may be a modified deoxyribonucleotide having a functional group capable of covalently binding to a functional group on the solid phase. The reaction mixture may further contain a chimeric oligonucleotide primer having a sequence substantially homologous to the nucleotide sequence of the nucleic acid as the template. The nucleic acid amplification method used for the first aspect may comprise:

(a) preparing a reaction mixture by mixing a nucleic acid as a template, a deoxyribonucleotide triphosphate, a DNA polymerase having a strand displacement activity, at least one primer and an RNase H, wherein the primer is a chimeric oligonucleotide primer that is substantially complementary to the nucleotide sequence of the nucleic acid as the template and contains deoxyribonucleotides and a ribonucleotide, the ribonucleotide being positioned at the 3' terminus or on the 3'-terminal side of the primer, wherein a part of the deoxyribonucleotides is a deoxyribonucleotide modified for immobilization onto a solid phase; and
(b) incubating the reaction mixture for a sufficient time to generate a reaction product.

Alternatively, the nucleic acid amplification method used for the first aspect may comprise:

(a) preparing a reaction mixture by mixing a nucleic acid as a template, deoxyribonucleotide triphosphates, a DNA polymerase having a strand displacement activity, at least one primer and an RNase H, wherein the primer is a chimeric oligonucleotide primer that is substantially complementary to the nucleotide sequence of the nucleic acid as the template and contains a deoxyribonucleotide and a ribonucleotide, the ribonucleotide being positioned at the 3' terminus or on the 3'-terminal side of the primer, wherein a part of the deoxyribonucleotide triphosphates is a decxyribonucleotide triphosphate modified for immobilization onto a solid phase; and
(b) incubating the reaction mixture for a sufficient time to generate a reaction product.

An amplified nucleic acid that contains or does not contain a ribonucleotide can be preferably prepared according to the first aspect.
Although it is not intended to limit the present invention, the RNase H used in the first aspect can be selected, for example, from the group consisting of an RNase H from Escherichia coli, an RNase H from a bacterium of genus Thermotoga, an RNase H from a bacterium of genus Thermus, an RNase H from a bacterium of genus Pyrococcus and an RNase H from a bacterium of genus Bacillus. Among these, type I RNase H from Escherichia coli, or type II RNase H from Pyrococcus furiosus or Pyrococcus horikoshii can be preferably used.
The second aspect of the present invention relates to a material having an immobilized amplified nucleic acid in which the amplified nucleic acid obtained according to the first aspect is immobilized and arrayed in a predefined region on a solid phase. In the material having an immobilized amplified nucleic acid, the amplified nucleic acid may be immobilized in a predefined region on a solid phase via a covalent bond.
The material having an immobilized amplified nucleic acid of the second aspect may be one which is obtained by immobilizing and arraying in a predefined region on a solid phase the amplified nucleic acid of the first aspect, which is obtained using a chimeric oligonucleotide primer that is substantially complementary to the nucleotide sequence of the nucleic acid as the template and contains a deoxyribonucleotide modified for immobilization onto a solid phase, and a chimeric oligonucleotide primer that is substantially homologous to the nucleotide sequence of the nucleic acid as the template and does not contain a deoxyribonucleotide modified for immobilization onto a solid phase; and then releasing only the strand extended from the chimeric oligonucleotide primer that does not contain a deoxyribonucleotide modified for immobilization onto a solid phase.
The material having an immobilized amplified nucleic acid of the second aspect may be one which is obtained by immobilizing via a covalent bond and arraying in a predefined region on a solid phase the amplified nucleic acid of the first aspect, which is obtained using a chimeric oligonucleotide primer that is substantially complementary to the nucleotide sequence of the nucleic acid as the template and contains a deoxyribonucleotide modified for immobilization onto a solid phase, and a chimeric oligonucleotide primer that is substantially homologous to the nucleotide sequence of the nucleic acid as the template and does not contain a deoxyribonucleotide modified for immobilization onto a solid phase; and then releasing only the strand extended from the chimeric oligonucleotide primer that does not contain a deoxyribonucleotide modified for immobilization onto a solid phase.
The third aspect of the present invention relates to a chimeric oligonucleotide primer represented by general formula below:

General formula: 5'-dNa-Nb-dNc-3'

(a: an integer of 11 or more; b: an integer of 1 or more; c: 0 or an integer of 1 or more; dN: deoxyribonucleotide and/or deoxyribonucleotide analog, wherein at least one of dNs is a deoxyribonucleotide modified for immobilization onto a solid phase; N: unmodified ribonucleotide and/or modified ribonucleotide, wherein some of dNs in dNa may be replaced by Ns).

According to the third aspect, c in the general formula may be 0, and the deoxyribonucleotide analog may be deoxyriboinosine nucleotide and the modified ribonucleotide may be ($\alpha$-S) ribonucleotide.

The fourth aspect of the present invention relate to a method for producing the amplified nucleic acid of the first aspect.

The fifth aspect of the present invention relates to a method for producing the material having an immobilized amplified nucleic acid of the second aspect.

Detailed Description of the Invention

As used herein, a deoxyribonucleotide (also referred to as a dN) refers to a nucleotide of which the sugar portion is composed of D-2-deoxyribose. The deoxyribonucleotides include, for example, ones having adenine, cytosine, guanine or thymine as the base portion. Furthermore, the deoxyribonucleotides also include a deoxyribonucleotide having a modified base such as 7-deazaguanosine and a deoxyribonucleotide analog such as deoxyinosine nucleotide.

As used herein, a deoxyribonucleotide modified for immobilization onto a solid phase refers to a deoxyribonucleotide or a deoxyribonucleotide analog having an added modification suitable for immobilization onto a solid phase. Examples thereof include a deoxyribonucleotide or a deoxyribonucleotide analog modified by adding a functional group such as an amino group, an aldehyde group or an acyl group or by adding biotin. The deoxynucleotides include such a deoxyribonucleotide modified for immobilization onto a solid phase.

As used herein, a ribonucleotide (also referred to as an N) refers to a nucleotide of which the sugar portion is composed of D-ribose. The ribonucleotides include ones having adenine, cytosine, guanine or uracil as the base portion. The ribonucleotides also include modified ribonucleotides such as a modified ribonucleotide in which the oxygen atom of the phosphate group at the $\alpha$-position is replaced by a sulfur atom (also referred to as an ($\alpha$-S) ribonucleotide or an ($\alpha$-S) N) or other derivatives.

As used herein, a chimeric oligonucleotide primer refers to a primer that contains a deoxyribonucleotide and a ribonucleotide. The primer may contain a deoxyribonucleotide analog and/or a modified deoxyribonucleotide and/or a modified ribonucleotide.

The chimeric oligonucleotide primers used in the present invention include any chimeric oligonucleotide primer that has a ribonucleotide being positioned at the 3' terminus or on the 3'-terminal side of the primer, can be used to extend a nucleic acid strand in the method of the present invention, can be cleaved with an endonuclease, and can be used to effect a strand displacement reaction.

As used herein, 3'-terminal side refers to a portion from the center to the 3' terminus of a nucleic acid such as a primer. Likewise, 5'-terminal side refers to a portion from the center to the 5' terminus of a nucleic acid.

As used herein, an endonuclease may be any one that acts on a double-stranded DNA generated by extending a DNA from the chimeric oligonucleotide primer which have been annealed to a nucleic acid as a template, and specifically cleaves it at a portion of the primer that contains a ribonucleotide.

As used herein, a DNA polymerase refers to an enzyme that synthesizes a DNA strand de novo using a DNA strand as a template. The DNA polymerases include naturally occurring DNA polymerases and variant enzymes having the above-mentioned activity. For example, such enzymes include a DNA polymerase having a strand displacement activity, a DNA polymerase lacking a 5'$\rightarrow$3' exonuclease activity and a DNA polymerase having a reverse transcriptase activity or an endonuclease activity.

Hereinafter, the present invention will be described in detail.

(1) Chimeric oligonucleotide primer used in the present invention

The primer used in the method of the present invention is a chimeric oligonucleotide primer that contains a deoxyribonucleotide and a ribonucleotide. Such primers also include an oligoribonucleotide primer that contains an unmodified deoxyribonucleotide and/or a modified deoxyribonucleotide and an unmodified ribonucleotide and/or a modified ribonucleotide. Furthermore, the primer may contain a deoxyribonucleotide analog.

A chimeric oligonucleotide primer used in the method of the present invention is a chimeric oligonucleotide primer that has a nucleotide sequence substantially complementary to a part of the nucleotide sequence of a nucleic acid as a template. It can extend a DNA strand from the 3' terminus. Furthermore, a ribonucleotide is positioned at the 3' terminus or on the 3'-terminal side of the chimeric oligonucleotide primer. The primer is usually designed such that it is complementary to a portion upstream of the region to be amplified, that is, a portion 3' to the nucleotide sequence corresponding to a region to be amplified in a nucleic acid as a template. As used herein, "a substantially complementary nucleotide sequence" means a nucleotide sequence that can anneal to a nucleic acid (DNA) as a template under reaction conditions used.

"A substantially homologous nucleotide sequence" refers to a nucleotide sequence so homologous to a nucleic acid (DNA) as a template that it can anneal to a complementary strand of the nucleic acid (DNA) as a template under reaction conditions used.

The chimeric oligonucleotide primer used in the method of the present invention may contain one or more modified ribonucleotide. As used herein, a ribonucleotide may be an unmodified ribonucleotide and/or a modified ribonucleotide that can be positioned at the 3' terminus or on the 3'-terminal side of a chimeric oligonucleotide primer and that is recognized by or cleaved with an endonuclease. The ribonucleotides include both of the unmodified ribonucleotide and the modified ribonucleotide as described above. An unmodified ribonucleotide, a modified ribonucleotide or a combination thereof can be used for the chimeric oligonucleotide primer of the present invention as long as it does not abolish the function of the primer. Examples of the modified ribonucleotides include, but are not limited to, an ($\alpha$-S) ribonucleotide in which the oxygen atom bound to the phosphate group is replaced by a sulfur atom, and a ribonucleotide in which the hydroxy group at the 2-position of the ribose is replaced by a methoxy group. Such a chimeric oligonucleotide primer containing a modified ribonucleotide can be produced by using, for example, an ($\alpha$-S) ribonucleotide triphosphate, which is prepared by a method using a sulfuration reaction reagent (Glen Research) as described in United States Patent No. 5,003,097, or a 2-OMe-RNA-CE phosphoramidite reagent (Glen Research).

The length of the chimeric oligonucleotide primer used in the method of the present invention is not specifically limited, but is preferably about 12 nucleotides to about 100 nucleotides, more preferably about 15 nucleotides to about 40 nucleotides. It is preferable that the nucleotide sequence of the chimeric oligonucleotide is substantially complementary to a nucleic acid as a template such that it anneals to the nucleic acid as the template under reaction conditions used. The primer contains a sequence recognized by an endonuclease, which is utilized in a step as described below, at the 3' terminus or on the 3'-terminal side.

The chimeric oligonucleotide primer used in the present invention has a structure in which an endonuclease recognizes or cleaves a DNA strand extended from the primer using a DNA polymerase (a primer-extended strand) at a site that contains a ribonucleotide, which ribonucleotide being positioned at the 3' terminus or on the 3'-terminal side of the chimeric oligonucleotide primer. Although it is not intended to limit the present invention, for example, when an RNase H acts on a double-stranded DNA generated by extending a DNA from a chimeric oligonucleotide primer represented by the general formula that has been annealed to a nucleic acid as a template, the chimeric oligonucleotide primer is cleaved at the ribonucleotide portion. A double-stranded DNA in which a nick is introduced between the oligonucleotide primer and the DNA strand synthesized by the extension is then generated. Then, a strand displacement reaction with a DNA polymerase proceeds from the nicked site. Thus, any chimeric oligonucleotide primer that can be used to extend a nucleic acid strand from the 3' terminus of the primer, that can be cleaved with an endonuclease, and with which a DNA polymerase can effect a strand displacement reaction can be used in the method of the present invention.

Furthermore, the chimeric oligonucleotide primer used in the method of the present invention may contain one or more modified deoxyribonucleotide(s) or deoxyribonucleotide analog(s) positioned 5' to the ribonucleotide position. That is, a modified deoxyribonucleotide or a deoxyribonucleotide analog can be contained in the chimeric oligonucleotide primer of the present invention as long as the function of the primer is not abolished. Plural types of the modified deoxyribonucleotides or the deoxyribonucleotide analogs can be used in combination. Examples of the modified deoxyribonucleotides include, but are not limited to, a modified deoxyribonucleotide having an incorporated amino group. Examples of the deoxyribonucleotide analogs include, but are not limited to, deoxyinosine nucleotide and a deoxyribonucleotide analog having a modified base such as 7-deazaguanine.

For example, a chimeric oligonucleotide primer represented by general formula below is preferably used as the chimeric oligonucleotide primer of the present invention as a primer, although it is not intended to limit the present invention:

General formula: 5'-dNa-Nb-dNc-3'

(a: an integer of 11 or more; b: an integer of 1 or more; c: 0 or an integer of 1 or more; dN: deoxyribonucleotide and/or deoxyribonucleotide analog, wherein at least one of dNs is a deoxyribonucleotide modified for immobilization onto a solid phase; N: unmodified ribonucleotide and/or modified ribonucleotide, wherein some of dNs in dNa may be replaced by Ns).

Although it is not intended to limit the present invention, for example, the chimeric oligonucleotide primer is in a form of 5'-DNA-RNA-DNA-3' if b is an integer of 1 or more and c is an integer of 1 or more.

In the chimeric oligonucleotide primer, c in the general formula may be 0, and the deoxyribonucleotide analog may be deoxyriboinosine nucleotide and the modified ribonucleotide may be ($\alpha$-S) ribonucleotide.

A nucleotide in the dNa portion and/or the dNc portion having a functional group (e.g., an amino group, an aldehyde group or an acyl group) or biotin being added can be preferably used for adding such a functional group or biotin used for immobilization onto a substrate. Although it is not intended to limit the present invention, a chimeric oligonucleotide primer in which an amino group is added to any one of the nucleotides in the dNa portion on the 5' side, a chimeric oligonucleotide primer in which an amino group is added to any one of the nucleotides in the dNc portion on the 3' side, and a chimeric oligonucleotide primer in which amino groups are added to any one of the nucleotides in the dNa portion on the 5' side and any one of the nucleotides in the dNc portion on the 3' side can be preferably used.

The chimeric oligonucleotide primer can be synthesized to have desired nucleotide sequence using, for example, the 394 type DNA synthesizer from Applied Biosystems Inc. (ABI) according to a phosphoramidite method. Alternatively, any methods including a phosphate triester method, an H-phosphonate method and a thiophosphonate method may be used to synthesize the chimeric oligonucleotide primer.

(2) Endonuclease used in the present invention

Any endonuclease that can act on a double-stranded DNA generated by DNA extension from the chimeric oligonucleotide primer as described above in (1) that has been annealed to a nucleic acid as a template and cleaves the extended strand to effect a strand displacement reaction may be used in the present invention. That is, the endonuclease is an enzyme that can generate a nick in the chimeric oligonucleotide primer portion of the double-stranded DNA. For example, ribonucleases can be used. Among these, endoribonuclease H (RNase H) that acts on an RNA portion of a double-stranded nucleic acid composed of a DNA and an RNA can be preferably used. Any ribonuclease that has the above-mentioned activities can be preferably used in the present invention, including mesophilic and heat-resistant ones. For example, an RNase H from E. coli, a heat-resistant ribonuclease such as an RNase H from a bacterium of genus Thermotoga, a bacterium of genus Thermus, a bacterium of genus Pyrococcus or a bacterium of genus Bacillus can be preferably used. A type I, II or III RNase H can be used in the method of the present invention.

Although it is not intended to limit the present invention, type II RNase H from Pyrococcus furiosus or Pyrococcus horikoshii as an exemplary RNase H from a bacterium of genus Pyrococcus specifically cleaves a chimeric double-stranded nucleic acid at a site 5' to an RNA at an RNA-DNA junction even if the number of RNA is one. The chimeric double-stranded nucleic acid is obtained by annealing of the chimeric oligonucleotide primer as described in (1) above to a substantially complementary nucleic acid and extension. The RNAs contained in the chimeric oligonucleotide primer as described in (1) above are cleaved off one by one from the 3' terminus in an ICAN reaction, and an ICAN product containing no RNA is finally obtained. The amplification product can be preferably used as a nucleic acid for immobilization for a material having an immobilized nucleic acid of the present invention.

(3) DNA polymerase used in the present invention

A DNA polymerase having a strand displacement activity on a DNA can be used in the present invention. Particularly, a DNA polymerase substantially lacking a 5'→3' exonuclease activity can be preferably used.

As used herein, "a strand displacement activity" refers to an activity that can effect a strand displacement, that is, that can proceed DNA duplication on the basis of a sequence of a nucleic acid as a template while displacing a DNA strand to release a complementary strand that has been annealed to the template strand.

Any DNA polymerases having the strand displacement activity can be used in the present invention. Examples thereof include variants of DNA polymerases lacking their 5'→3' exonuclease activities derived from thermophilic bacteria of genus Bacillus such as Bacillus caldotenax (hereinafter referred to as B. ca) and Bacillus stearothermophilus (hereinafter referred to as B. st), as well as large fragment (Klenow fragment) of DNA polymerase I from Escherichia coli (E. coli). Both of mesophilic and heat-resistant DNA polymerases can be preferably used in the present invention.

B. ca is a thermophilic bacterium having an optimal growth temperature of about 70°C. Bca DNA polymerase from this bacterium is known to have a DNA-dependent DNA polymerase activity, an RNA-dependent DNA polymerase activity (a reverse transcription activity), a 5'→3' exonuclease activity and a 3'→5' exonuclease activity. The enzyme may be either an enzyme purified from its original source or a recombinant protein produced by using genetic engineering techniques. The enzyme may be subjected to modification such as substitution, deletion, addition or insertion by using genetic engineering techniques or other means. Examples of such enzymes include BcaBEST DNA polymerase (Takara Shuzo), which is Bca DNA polymerase lacking its 5'→3' exonuclease activity.

It is known that some DNA polymerases have an endonuclease activity such as an RNase H activity under specific conditions. Such a DNA polymerase can be used in the method of the present invention. In one aspect, the DNA polymerase may be used under conditions that allow the RNase H activity to express, e.g., in the presence of $Mn^{2+}$. In this case, the method of the present invention can be conducted without the addition of an RNase H. The present inventors have demonstrated that the Bca DNA polymerase exhibits an RNase H activity in a buffer containing $Mn^{2+}$ for the first time, and that the nucleic acid amplification method of the present invention can be carried out in a reaction mixture containing no enzyme other than the Bca DNA polymerase. The above-mentioned aspect is not limited to the use of the Bca DNA polymerase. DNA polymerases that are known to have an RNase H activity such as Tth DNA polymerase from Thermus thermophilus can be used in the present invention.

(4) Deoxyribonucleotide triphosphate used in the present invention

dNTPs used for the PCR method or the like (a mixture of dATP, dGTP, dCTP and dTTP) can be preferably used according to the present invention as deoxyribonucleotide triphosphates that serve as substrates for an extension reaction. The dNTPs may contain a dNTP analog such as 7-deaza-dGTP as long as it serves as a substrate for the DNA polymerase used. Also, a deoxynucleotide triphosphate modified for immobilization of an ICAN product obtained by an ICAN reaction onto a solid phase may be contained as long as it serves as a substrate for the DNA polymerase used. Examples thereof include Aminosllyl dUTP and biotin-labeled deoxynucleotide (both from Sigma).

(5) Nucleic acid amplification method of the present invention

The nucleic acid amplification method of the present invention can be accomplished by conducting a reaction using at least one oligonucleotide primer as described in (1) above in combination with the endonuclease as described in (2) above, the DNA polymerase as described in (3) above and the deoxyribonucleotide triphosphate as described in (4) above.

About 100% of the primer used in a reaction according to the ICAN method is utilized. The amount of amplified region of interest can be several times higher than that in a conventional amplification reaction such as PCR. Furthermore, the method of the present invention does not require reaction equipment that can adjust a temperature over time. Therefore, an amplification reaction can be conducted in a large volume of reaction mixture.

The nucleic acid (DNA or RNA) used as a template in the method of the present invention may be prepared or isolated from any sample that may contain the nucleic acid. Alternatively, the sample may be used directly in the nucleic acid amplification reaction according to the present invention. Examples of the samples that may contain the nucleic acid include, but are not limited to, samples from organisms such as a whole blood, a serum, a buffy coat, a urine, feces, a cerebrospinal fluid, a seminal fluid, a saliva, a tissue (e.g., a cancerous tissue or a lymph node) and a cell culture (e.g., a mammalian cell culture or a bacterial cell culture), samples that contain a nucleic acid such as a viroid, a virus, a bacterium, a fungi, a yeast, a plant and an animal, samples suspected to be contaminated or infected with a microorganism such as a virus or a bacterium (e.g., a food or a biological formulation), and samples that may contain an organism such as a soil and a waste water. The sample may be a preparation containing a nucleic acid obtained by processing the above-mentioned samples according to a known method. Examples of the preparations that can be used in the present invention include a cell destruction product or a sample obtained by fractionating the product, the nucleic acid in the sample, or a sample in which specific nucleic acid molecules such as mRNAs are enriched. Furthermore, a nucleic acid such as a DNA or an RNA obtained amplifying a nucleic acid contained in the sample by a known method can be preferably used.

The preparation containing a nucleic acid can be prepared from the above-mentioned materials by using, for example, lysis with a detergent, sonication, shaking/stirring using glass beads or a French press, without limitation. In some cases, it is advantageous to further process the preparation to purify the nucleic acid (e.g., in case where an endogenous nuclease exists). In such cases, the nucleic acid is purified by a know method such as phenol extraction, chromatography, ion exchange, gel electrophoresis or density-gradient centrifugation.

If it is desired to amplify a nucleic acid having a sequence derived from an RNA, the method of the present invention may be conducted using as a template a cDNA synthesized by a reverse transcription reaction that uses the RNA as a template. Any RNA for which one can make a primer to be used in a reverse transcription reaction can be applied to the method of the present invention, including total RNA in a sample, RNA molecules such as mRNA, tRNA and rRNA as well as specific RNA molecular species.

Any primer that anneals to an RNA as a template under reaction conditions used can be used in the reverse transcription reaction. The primer may be a primer having a nucleotide sequence that is complementary to a specific RNA as a template (a specific primer), an oligo-dT (deoxythymine) primer and a primer having a random sequence (a random primer). In view of specific annealing, the length of the primer for reverse transcription is preferably 6 nucleotides or more, more preferably 9 nucleotides or more. In view of oligonucleotide synthesis, the length is preferably 100 nucleotides or less, more preferably 30 nucleotides or less. A chimeric oligonucleotide primer can be used as a primer for reverse transcription. The chimeric oligonucleotide primer can also be utilized as a primer for a strand displacement reaction in the nucleic acid amplification method according to the present invention using a cDNA obtained after reverse transcription as a template. Such a primer may be any one that has the properties as described above in (1) and that can be used in a reverse transcription reaction from an RNA.

Any enzyme that has an activity of synthesizing a cDNA using an RNA as a template can be used in the reverse transcription reaction. Examples thereof include reverse transcriptases originating from various sources such as avian myeloblastosis virus-derived reverse transcriptase (AMV RTase), Molony murine leukemia virus-derived reverse transcriptase (MMLV RTase) and Rous-associated virus 2 reverse transcriptase (RAV-2 RTase). In addition, a DNA polymerase that also has a reverse transcription activity can be used. An enzyme having a reverse transcription activity at a high temperature such as a DNA polymerase from a bacterium of genus Thermus (e.g., Tth DNA polymerase) and a DNA polymerase from a thermophilic bacterium of genus Bacillus is preferable for the present invention. For example, DNA polymerases from thermophilic bacteria of genus Bacillus such as a DNA polymerase from B. st (Bst DNA polymerase) and Bca DNA polymerase are preferable, although it is not intended to limit the present invention. For example, Bca DNA polymerase does not require a manganese ion for the reverse transcription reaction. Furthermore, it can synthesize a cDNA while suppressing the formation of a secondary structure of an RNA as a template under high-temperature conditions. Both a naturally occurring one and a variant of the enzyme having a reverse transcriptase activity can be used as long as they have the activity.

In another aspect, after duplicating a DNA or an RNA containing a nucleotide sequence to be amplified beforehand, the duplicated product may be used as a nucleic acid as a template in the method for obtaining a nucleic acid amplified according to the present invention. Examples of the methods for duplication include, but are not limited to, a method in which an appropriate host is transformed with a vector into which the nucleic acid fragment containing the nucleotide sequence to be amplified is inserted, the resulting transformant is cultured, the vector into which the nucleic acid fragment containing the nucleotide sequence to be amplified is inserted is extracted therefrom and used. Any vectors can be used as long as they are stably replicated in the host. For example, pUC series, pBluescript series, pGEM series, cosmid type vectors and phage type vectors are preferably used. Any hosts can be used as long as they can maintain the vectors used. For example, Escherichia coli, which is readily cultured, is exemplified.

In another aspect of the duplication method, after an RNA having a nucleotide sequence to be amplified is transcribed using an RNA polymerase and a nucleic acid fragment containing the nucleotide sequence as a template, the RNA may be used as a template for the method of the present invention directly or after converting it into a cDNA by reverse transcription reaction. The nucleic acid fragment containing the nucleotide sequence to be amplified is not limited to a specific one as long as it has a promoter sequence for an RNA polymerase. It may be inserted into a vector having a promoter sequence for an RNA polymerase, ligated with an adapter or a cassette having a promoter sequence for an RNA polymerase at its end or enzymatically synthesized using a primer having a promoter sequence for an RNA polymerase and an appropriate template. Thus, a nucleic acid fragment containing a nucleotide sequence to be amplified can be duplicated or amplified in a form of an RNA using a promoter sequence for an RNA polymerase being positioned as described above. Any vectors can be used as long as they have promoter sequences for RNA polymerases. For example, pUC series, pBluescript series, pGEM series, cosmid type vectors and phage type vectors are preferably used. The vector can be preferably used in its circular form or after being linearized. Any RNA polymerases can be used for the duplication or amplification method. For example, SP6 RNA polymerase, T7 RNA polymerase, T3 RNA polymerase or the like can be preferably used.

By preparing a nucleic acid as a template in a form as described above which is to be used for obtaining a nucleic acid amplified according to the present invention, a limited number of oligonucleotide primers as described in (1) above can be used to obtain a variety of amplified nucleic acids. That is, a variety of DNA fragments such as those used for immobilization onto a DNA chip can be obtained in large quantities as nucleic acids amplified according to the present invention.

The suitable length of the template is one that provides a sufficient binding of the oligonucleotide primer sequence as described in (1) above due to the presence of the whole target sequence or at least a sufficient part of the target sequence in the fragment.

Both a double-stranded DNA such as a genomic DNA or an amplified nucleic acid fragment and a single-stranded DNA such as a cDNA prepared by a reverse transcription reaction from a total RNA or an mRNA can be preferably used as a template DNA for obtaining a nucleic acid amplified according to the present invention. The double-stranded DNA may be denatured into single-stranded DNAs. Alternatively, an RNase H that make the denaturation step unnecessary may be used in combination. Such an RNase H is exemplified by P. fu or P. ho type II RNase H.

Although it is not intended to limit the present invention, if a DNA as a template to be used for obtaining a nucleic acid amplified according to the present invention is a double-stranded DNA, the DNA can be denatured into single-stranded DNAs to allow a primer to bind to the DNA strand as the template. Incubating the double-stranded DNA at a temperature at which it is denatured (e.g., about 95°C) is preferable for the denaturation. Other processes include one in which an elevated pH is used. In this case, the pH should be lowered upon the amplification reaction in order to allow an oligonucleotide primer to bind to a target. A nucleic acid is successively amplified under isothermal conditions after denaturing a double-stranded DNA into single-stranded DNAs or, if an RNA is used as a template, preparing a cDNA (a single-stranded DNA) by a reverse transcription reaction. "Successively" means that a reaction proceeds without a change in the reaction temperature or the composition of the reaction mixture. As used herein, "isothermal" means conditions of a substantially constant temperature under which an enzyme and a nucleic acid strand function in each step.

The nucleic acid amplification reaction for obtaining a nucleic acid amplified according to the present invention may be conducted at a normal temperature (e.g., 37°C) by using a mesophilic DNA polymerase such as Klenow fragment. It can also be conducted at a high temperature (e.g., 50°C or higher, or 60°C or higher) by using heat-resistant enzymes (an endonuclease and a DNA polymerase). In this case, non-specific annealing of a primer is suppressed, resulting in increase in the specificity of DNA amplification. Furthermore, the problem of forming secondary structure of a DNA as a template is solved, resulting in increase in the ability of extension of a DNA polymerase. A reverse transcription reaction and the nucleic acid amplification can be successively conducted in the method. In this case, a DNA having a sequence derived from an RNA can be amplified by combining the use of a reverse transcriptase with the above-mentioned reaction or by using a DNA polymerase having a reverse transcription activity.

In each aspect of the reaction for obtaining a nucleic acid amplified according to the present invention, preferably, a chimeric oligonucleotide primer that is complementary to a single-stranded DNA as a template is first annealed to the DNA, although it is not intended to limit the present invention. A DNA that is complementary to the DNA as the template (a primer-extended strand) is then extended along the remained sequence of the DNA as the template from the 3' terminus of the primer by the action of a DNA polymerase to synthesize a double-stranded DNA. An endonuclease acts on the double-stranded DNA and begins to extend a DNA from the primer portion of the primer-extended strand de novo. The endonuclease acts as a nicking enzyme that introduces a nick into the double-stranded DNA or it alters the structure of the double-stranded DNA composed of the chimeric oligonucleotide primer and the DNA as the template, although the present invention is not restricted by a theory. A DNA polymerase having a strand displacement activity re-extends a DNA strand from the 3' terminus of the nick introduced in the double-stranded DNA to generate a new primer-extended strand while releasing the DNA downstream from the 3' terminus of the nick.

The nucleic acid amplification method for obtaining a nucleic acid amplified according to the present invention can be carried out using two primers, i.e., a chimeric oligonucleotide primer that is complementary to a nucleic acid as a template and another chimeric oligonucleotide primer that is complementary to a displaced strand. In this case, one primer binds to a DNA strand as a template to cause a strand displacement reaction, whereas another primer binds to a displaced strand released as a result of the strand displacement reaction to initiate another strand displacement reaction. It is clear that a reaction product with one primer can function as a template for another primer if this aspect is used. Thus, the amount of amplification product increases in a non-linear manner as the amount of the template increases. A nucleic acid amplified according to the present invention can be obtained by using a deoxyribonucleotide modified for immobilization onto a solid phase as a part of chimeric oligonucleotide.

When the nucleic acid amplification method for obtaining a nucleic acid amplified according to the present invention is conducted using a double-stranded DNA as a template, both strands can serve as templates in the amplification reaction by using chimeric oligonucleotide primers that anneal to the respective two strands. If the reaction is initiated after denaturing the double-stranded DNA, a chimeric oligonucleotide primer, four deoxyribonucleotide triphosphates (dNTPs), a DNA polymerase and an endonuclease are added to a reaction mixture before or after the double-stranded DNA is denatured. If heat treatment is used for denaturing the double-stranded DNA and a heat-resistant enzyme is not used, it is preferable to add the enzyme after the denaturation. A nucleic acid amplified according to the present invention can be obtained using a deoxyribonucleotide triphosphate modified for immobilization onto a solid phase as a part of deoxyribonucleotide triphosphates.

In the aspect of the nucleic acid amplification method for obtaining a nucleic acid amplified according to the present invention in which a double-stranded DNA as a template and two chimeric oligonucleotide primers are used, switching of templates may occur among the template-extended strand intermediates during the extension reaction from the primers to generate a double-stranded nucleic acid consisting of the synthesized primer-extended strands being annealed each other, although it depends on the reaction conditions or the like. The double-stranded nucleic acid has chimeric cligonucleotide primers at both ends. Then, a reaction of extending complementary strands comprising strand displacement can be initiated from both of the ends again. As a result of the reaction, an amplification product having the primer sequence at one end is generated. Furthermore, if switching of templates occurs during the reaction, a double-stranded nucleic acid similar to one that described above is generated again.

A DNA polymerase capable of effecting the template switching reaction during the strand displacement reaction can be preferably used for obtaining a nucleic acid amplified according to the present invention. For example, a variant enzyme of Bca DNA polymerase lacking a 5'→3' exonuclease activity is preferably used in particular. Such an enzyme is commercially available as BcaBEST DNA polymerase (Takara Shuzo). It can also be prepared from Escherichia coli HB101/pU1205 (FERM BP-3720) which contains the gene for the enzyme according to the method as described in Japanese Patent No. 2978001. Escherichia coli HB101/pU1205 (FERM BP-3720) has been deposited at International Patent Organism Depositary, National Institute of Advanced Industrial Science and Technology, AIST Tsukuba Central 6, 1-1, Higashi 1-chome, Tsukuba-shi, Ibaraki 305-8566, Japan since May 10, 1991 (date of original deposit).

In the amplification method for obtaining a nucleic acid amplified according to the present invention, the chimeric oligonucleotide primer-extended strand may be cleaved at a site that contains the ribonucleotide such that the 5' fragment (primer portion) resulting from the cleavage does not contain the ribonucleotide. A primer-extended strand extended from the thus generated primer portion is no longer cleaved with an endonuclease. As a result, an amplification product having the primer sequence at its end is generated.

As described above, an amplification product without the primer sequence and a product having the primer sequence(s) at one or both of the ends may be generated in the nucleic acid amplification method for obtaining a nucleic acid amplified according to the present invention. These products are included in the nucleic acids amplified according to the present invention.

The DNA polymerase used in the method for obtaining a nucleic acid amplified according to the present invention should synthesize an extended strand from the 3' terminus of the primer portion towards the downstream while displacing a previously extended DNA strand. It is important that the DNA polymerase should not exhibit a 5'→3' exonuclease activity that may digest the displaced strand. For example, Klenow fragment, which is an exonuclease-deficient variant of DNA polymerase I from Escherichia coli, a similar fragment derived from Bst DNA polymerase (New England Biolabs), and BcaBEST DNA polymerase from B. ca (Takara Shuzo) are useful as such a DNA polymerase. Sequenase 1.0 and Sequenase 2.0 (United States Biochemical), and T5 DNA polymerase and φ29 DNA polymerase as described in Gene, 97:13-19 (1991) can also be used. A polymerase that normally has a 5'→3' exonuclease activity can be used in the DNA synthesis method for obtaining a nucleic acid amplified according to the present invention if addition of an appropriate inhibitor can inhibit the activity.

The nucleic acid amplification method for obtaining a nucleic acid amplified according to the present invention may be conducted at varying temperatures or it may be conducted isothermally. Varying temperatures means that the reaction temperatures are changed for respective steps such that the change does not interfere with the reactions in the steps. Specifically, varying temperatures refers to change in temperature to that suitable for, for example, each of annealing of a primer, synthesis reaction of a complementary strand, nicking of a complementary strand and a displacement reaction.

On the other hand, isothermal means that the reaction temperature for each step is unchanged and each step is conducted at a substantially constant temperature. It is natural to select the temperature to optimize the reaction conditions in both cases.

One feature of the amplification method for obtaining a nucleic acid amplified according to the present invention is that the method does not require adjusting the temperature up and down during the nucleic acid synthesis. Many of conventional nucleic acid amplification methods require adjusting the temperature up and down to dissociate a target from a synthesized strand. These methods require special reaction equipment such as a thermal cycler for this purpose. However, the method of the present invention can be conducted only using equipment that can keep a constant temperature. As described above, the method of the present invention can be conducted at a single temperature. Preferably, it is conducted by selecting the reaction temperature and the stringency level such that non-specific annealing of a primer is reduced and such that the primer specifically anneals to a nucleic acid as a template. Although it is not intended to limit the present invention, the method of the present invention can be conducted under high-temperature conditions by using a heat-resistant enzyme as described above. In addition, it is preferable to conduct the method of the present invention at an appropriate temperature for sufficiently retaining the activity of the enzyme used in order to maintain the reaction efficiency at high level. Thus, the reaction temperature is preferably about 20°C to about 80°C, more preferably about 30°C to about 75°C, most preferably about 50°C to about 70°C although it varies depending on the enzyme used. When the reaction is conducted under high-temperature conditions in particular, it is preferable to use a longer primer than that for a reaction at a normal temperature. The sequence and the length of the primer appropriate for the reaction temperature may be determined, for example, with reference to its Tm value. Alternatively, a commercially available software for designing a primer such as OLIGO™ Primer Analysis software (Takara Shuzo) may be used. For example, when a reaction temperature of 55°C to 60°C or 65°C is used, the primer used for the method may be, for example, without limitation, 12-100 nucleotides in length, preferably 14-50 nucleotides in length, more preferably 15-40 nucleotides in length. An example of effects brought by the elevated reaction temperature is the solution of a problem of forming secondary structure of a DNA as a template. The elevated reaction temperature enables amplification of a desired nucleic acid even if a nucleic acid having a high GC content is used as a template. Furthermore, it is similarly effective in amplifying a region of a long chain length. Such effect is observed in a range between about 60 bp and about 20 kbp, specifically between about 110 bp and about 4.3 kbp, more specifically about 130 bp and about 1500 bp.

The amplification efficiency can be increased by adjusting the reaction temperature in accordance with the GC content of the nucleic acid as the template. For example, if a nucleic acid having a low GC content is used as a template, the amplification reaction can be conducted at 50 to 55°C, although the temperature depends on the chain length to be amplified and the Tm value of the primer.

Use of a DNA polymerase having a reverse transcriptase activity (e.g., BcaBEST DNA polymerase) in the method can make the amplification of a nucleic acid from an RNA, which comprises a step of preparing a cDNA from an RNA (a reverse transcription reaction), be conveniently conducted. Alternatively, a product obtained by independently conducting a step of preparing a cDNA from an RNA, i.e., a cDNA, can be used in the method for obtaining a nucleic acid amplified according to the present invention as the DNA as a template.

A region of a nucleic acid sequence of interest can be amplified by conducting the method for obtaining a nucleic acid amplified according to the present invention in a buffer of phosphoric acid, Bicine, Tricine, HEPES, phosphate or tris.

Furthermore, the nucleic acid amplification methods does not require the use of reaction equipment that can adjust a temperature with the passage of time. Therefore, an amplification reaction can be conducted in a large volume of reaction mixture. Thus, a nucleic acid amplified according to the present invention can be industrially produced in large quantities.

The utilization efficiency of the primer used in the amplification method for obtaining a nucleic acid amplified according to the present invention is about 100%, which may be 5- to 10-fold higher than that in a conventional method such as the PCR.

(6) Method for producing amplified nucleic acid of the present invention

As described above, a nucleic acid amplified according to the present invention can be obtained by preparing a reaction mixture by mixing a nucleic acid as a template, deoxyribonucleotide triphosphates including a deoxyribonucleotide triphosphate modified for immobilization onto a solid phase, a DNA polymerase having a strand displacement activity, at least one primer and an RNase H; and incubating the reaction mixture for a sufficient time to generate a reaction product.

In other words, there is no specific limitation concerning the deoxyribonucleotide triphosphate modified for immobilization onto a solid phase as long as it can be incorporated as a substrate using a DNA polymerase used in the method of the present invention. One having a functional group capable of immobilizing a nucleic acid prepared according to the method of the present invention can be preferably used as the modified deoxyribonucleotide triphosphate. Examples of the modified deoxyribonucleotide phosphates include, but are not limited to, a modified deoxyribonucleotide phosphate having a hydroxyl group, an amino group, a thiol group, an aldehyde group, a carboxyl group, an epoxy group or an acyl group, and a biotin-labeled deoxyribonucleotide triphosphate. Aminoallyl dUTP can be preferably used as such a modified deoxyribonucleotide triphosphate.

In another embodiment of the present invention, the method for producing an amplified nucleic acid may be characterized by incorporating a deoxyribonucleotide modified for immobilization onto a solid phase by using a chimeric oligonucleotide primer containing a deoxyribonucleotide modified for immobilization onto a solid phase. Thus, an exemplary method for producing an amplified nucleic acid comprises preparing a reaction mixture by mixing a nucleic acid as a template, a deoxyribonucleotide triphosphate, a DNA polymerase having a strand displacement activity, at least one primer and an RNase H; and incubating the reaction mixture for a sufficient time to generate a reaction product. In this case, the primer is a chimeric oligonucleotide primer that is substantially complementary to the nucleotide sequence of the nucleic acid as the template and contains deoxyribonucleotides and a ribonucleotide, the ribonucleotide being positioned at the 3' terminus or on the 3'-terminal side of the primer, wherein a part of the deoxyribonucleotides is a deoxyribonucleotide modified for immobilization onto a solid phase.

Although it is not intended to limit the present invention, for example, the chimeric oligonucleotide primer to be used in the method may have a functional group for immobilization onto a solid phase on the 5'-terminal side and/or the 3'-terminal side of the primer. In this case, the chimeric oligonucleotide primer may have a spacer between the functional group and the 5'-terminal side and/or the 3'-terminal side of the primer. There is no specific limitation concerning the functional group as long as it is capable of immobilizing onto a solid phase and does not inhibit the amplification according to the method of the present invention. One having a functional group capable of immobilizing a nucleic acid prepared according to the method of the present invention can be preferably used as such a modified primer. Although it is not intended to limit the present invention, examples thereof include a modified primer having a hydroxyl group, an amino group, a thiol group, an aldehyde group, a carboxyl group, an epoxy group or an acyl group at the 5' terminus. A 5'-Amino Link primer obtained using a reagent for introducing an amino group (Perkin Elmer) can be preferably used as such a modified primer.

Since the PCR method requires changing the temperature of the reaction mixture over time, the reaction volume is limited to one in which the temperature can be controlled (usually, 200 μl or less). Therefore, it is difficult to scale up the volume. On the other hand, there is no such limitation in the method of the present invention. A large amount of nucleic acid can be produced by increasing the volume of the reaction mixture. In the method of the present invention, a number of complementary strand molecules are synthesized from one template molecule. Furthermore, nucleic acids can be synthesized using these complementary strand molecules as templates. Thus, a desired nucleic acid can be efficiently produced in large quantities by suitably selecting the template and the primer. Additionally, the fact that, unlike the PCR, the method of the present invention does not require special equipment or a complicated temperature change makes it advantageous in view of the cost of equipment and energy. Therefore, the method is an excellent industrial method for producing a nucleic acid in large quantities.

The production method of the present invention is useful as a method for supplying a variety of DNA fragments in large quantities, such as those to be immobilized onto a DNA chip. Specifically, DNA fragments can be obtained in large quantities in simple reaction steps in one embodiment.

(7) Material having immobilized amplified nucleic acid of the present invention

Although it is not intended to limit the present invention, a DNA chip exemplifies a material having an immobilized nucleic acid. A DNA chip (also referred to as a DNA microarray or a DNA array) is a material having an immobilized nucleic acid in which various fragments of genes or DNAs are arrayed and immobilized in a predefined region or at a predefined position on a solid substrate such as a slide glass. The term a predefined region, a predefined position or a predetermined region means that the amplified nucleic acid immobilized in the region or at the position is known. In this case, the amplified nucleic acid immobilized at the position is known whether the nucleotide sequence of the amplified nucleic acid is known or unknown.

The DNA chip is used for examining the presence of a nucleic acid in a nucleic acid sample that has a sequence complementary to an arrayed and immobilized DNA in a predefined region on the DNA chip. The examination is carried out by contacting the DNA chip with the nucleic acid sample prepared from a sample, preferably a labeled nucleic acid sample, for hybridization. Since the DNA chip can be used to detect or quantify a number of nucleic acids in a sample in one procedure, it is a very useful means that dramatically promotes the analysis of gene expression, or the analysis of a mutation or polymorphism. A DNA chip in which a double-stranded nucleic acid is arrayed and immobilized in a predefined region is used for hybridization after it is subjected to appropriate denaturation. A DNA chip in which a single-stranded DNA complementary to a target nucleic acid to be detected is arrayed and immobilized in a predefined region is particularly preferable for the detection of a target nucleic acid.

Any insoluble substrate can be used as a substrate onto which the thus obtained DNA is arrayed and immobilized in a predefined region, but a plate-shaped substrate made from glass or plastic, and a membrane-shaped substrate made from nitrocellulose or nylon are preferably used. A known method for immobilizing a nucleic acid can be used for the immobilization. The DNA may be directly immobilized onto a substrate. Alternatively, the DNA may be immobilized through a suitable linker or after ligating plural DNA molecules.

Although it is not intended to limit the present invention, if a glass substrate is to be used, it is used after subjecting it to coating because glass itself is less charged and binds weakly to a DNA. Poly-L-lysine-coating, silylene-coating, silanization, glutaraldehyde-coating, amination or the like may be used for coating, without limitation. A DNA can be immobilized onto a glass substrate coated as described above by electrostatic force.

The nucleic acid amplified according to the present invention contains a deoxyribonucleotide modified for immobilization onto a solid phase. Therefore, it can be immobilized onto a substrate in a manner different from the immobilization onto a glass substrate by electrostatic force as described above, for example. Specifically, it is possible to utilize a covalent bond resulting from a chemical reaction between a functional group coated on a glass substrate and a modified deoxyribonucleotide contained in a nucleic acid amplified according to the present invention for immobilization.

Utilizing such a covalent binding, a material having an immobilized nucleic acid in which a single-stranded DNA is bound to a substrate can be obtained as follows. A nucleic acid amplified according to the present invention is prepared using a chimeric oligonucleotide primer that is substantially complementary to a nucleotide sequence of a nucleic acid as a template and contains a deoxyribonucleotide modified for immobilization onto a solid phase and a chimeric oligonucleotide primer that is substantially homologous to a nucleotide sequence of a nucleic acid as a template and does not contain a deoxyribonucleotide modified for immobilization onto a solid phase. The amplified nucleic acid is immobilized and arrayed in a predefined region in a glass substrate coated with a functional group by generating a covalent bond by a chemical reaction. Only the extended strand that does not contain the modified deoxyribonucleotide primer is then released by denaturation.

For example, since a ribonucleotide portion remains in a nucleic acid amplified according to the present invention obtained using RNase H from Escherichia coli, the bond of the ribonucleotide may be cleaved upon the release of only the extended strand that does not contain the modified deoxyribonucleotide primer after denaturation. However, the denaturation can be carried out such that a hydrogen bond in a double-stranded nucleic acid is cleaved, but the bond of the ribonucleotide in the same strand is not cleaved by carrying out the denaturation under the denaturation conditions according to the present invention as described below. As a result, a material having an immobilized nucleic acid in which a single-stranded nucleic acid containing a ribonucleotide is bound onto a substrate utilizing a covalent bond due to a modified deoxyribonucleotide in the amplified nucleic acid can be obtained.

Although there is no specific limitation concerning the conditions for denaturing the amplified nucleic acid, denaturation at a temperature of 10°C to 65°C in a solution containing an alkali at a concentration of 0.1 N to 2.0 N for 5 minutes to 30 minutes is preferable. In particular, if an amplified nucleic acid is immobilized onto a solid phase at its end, denaturation in 0.2 N NaOH at 20°C for 5 minutes, or in 0.5 N NaOH at 20°C for 5 minutes is preferable.

For example, if an amplified nucleic acid is immobilized onto a solid phase utilizing a modified deoxyribonucleotide in the amplified nucleic acid, denaturation in 0.2 N NaOH at 60°C for 30 minutes is preferable.

If the nucleic acid amplified according to the present invention is obtained using type II RNase H from Pyrococcus furiosus or Pyrococcus horikoshii, an amplified nucleic acid that does not contain a ribonucleotide portion can be obtained due to the property or the enzyme. Thus, in this case, a material having an immobilized nucleic acid in which a single-stranded nucleic acid is bound to a substrate can be obtained even if denaturation conditions stronger than those under which a hydrogen bond in a double-stranded nucleic acid is cleaved, but the bond of the ribonucleotide in the same strand is not cleaved are used.

For example, denaturation of such an amplified nucleic acid in 0.2 N NaOH at 60°C for 30 minutes is preferable.

A method other than the above-mentioned alkali denaturation such as heat denaturation may be carried out, although it is not intended to limit the present invention. For example, heat denaturation is carried out at 95°C for 3 minutes.

A material having an immobilized amplified nucleic acid of the present invention in which a single-stranded nucleic acid is immobilized onto a solid phase at its end or at a site of a modified nucleic acid contained in the nucleic acid can be obtained using a type II RNase H from Escherichia coli, Pyrococcus furiosus or Pyrococcus horikoshii.

If a double-stranded nucleic acid is arrayed and immobilized in a predetermined and predefined region in a material having an immobilized nucleic acid, the material is used for hybridization after subjecting it to appropriate denaturation. On the other hand, since a single-stranded DNA complementary to a target nucleic acid to be detected is arrayed and immobilized in a predefined region on the material of the present invention, it can be utilized for detecting a target nucleic acid without such denaturation. Thus, a DNA chip prepared using a product amplified according to the present invention in which a single-stranded DNA is bound to a substrate can be used detect a target nucleic acid more conveniently, with higher sensitivity and with higher reproducibility than a conventional one.

In addition, a chimeric oligonucleotide primer in a form of 5'-DNA-RNA-DNA-3' can be used according to the present invention. In this case, the utilization rate of a nucleic acid to be immobilized can be increased by introducing a functional group for immobilization onto a substrate into any one of nucleotides in a DNA portion on the 5' terminal side and/or the 3'-terminal side. In other words, loss of utilization of a nucleic acid to be immobilized can be decreased.

## Examples

The following Examples illustrate the present invention in more detail, but are not to be construed to limit the scope thereof.

### Referential Example 1

(1) Preparation of genomic DNA from Pyrococcus furiosus

2 L of a medium containing 1% Tryptone (Difco Laboratories), 0.5% yeast extract (Difco Laboratories), 1% soluble starch (Nacalai Tesque), 3.5% Jamarin S Solid (Jamarin Laboratory), 0.5% Jamarin S Liquid (Jamarin Laboratory), 0.003% $MgSO_4$, 0.001% NaCl 0.0001% $FeSO_4 \cdot 7H_2O$, 0.0001% $CoSO_4$, 0.0001% $CaCl_2 \cdot 7H_2O$, 0.0001% $ZnSO_4$, 0.1 ppm $CuSO_4 \cdot 5H_2O$ 0.1 ppm $KAl(SO_4)_2$, 0.1 ppm $H_3BO_4$, 0.1 ppm $Na_2MoO_4 \cdot 2H_2O$ and 0.25 ppm $NiCl_2 \cdot 6H_2O$ was placed in a 2-L medium bottle, sterilized at 120°C for 20 minutes, bubbled with nitrogen gas to remove dissolved oxygen, then Pyrococcus furiosus (purchased from Deutsche Sammlung von Mikroorganismen; DSM3638) was inoculated into the medium and cultured at 95°C for 16 hours without shaking. After cultivation, cells were collected by centrifugation. The resulting cells were then suspended in 4 ml of 25% sucrose, 50 mM tris-HCl (pH 8.0). 0.4 ml of 10 mg/ml lysozyme chloride (Nacalai Tesque) in water was added thereto. The mixture was reacted at 20°C for 1 hour. After reaction, 24 ml of a mixture containing 150 mM NaCl, 1 mM EDTA and 20 mM tris-HCl (pH 8.0), 0.2 ml of 20 mg/ml proteinase K (Takara Shuzo) and 2 ml of 10% aqueous solution of sodium lauryl sulfate were added to the reaction mixture. The mixture was incubated at 37°C for 1 hour. After reaction, the mixture was subjected to phenol-chloroform extraction followed by ethanol precipitation to prepare about 1 mg of genomic DNA.

(2) Cloning of RNase HII gene

The entire genomic sequence of Pyrococcus horikoshii was published [DNA Research, 5:55-76 (1998)]. The existence of one gene encoding a homologue of RNase HII (PH1650) in the genome was known (SEQ ID NO:1, the home page of National Institute of Technology and Evaluation, Ministry of Economy, Trade and Industry: http://www.nite.go.jp/).

Homology between the PH1650 gene and the partially published genomic sequence of Pyrococcus furiosus (the home page of University of Utah, Utah Genome Center: http://www.genome.utah.edu/sequence.html) was searched. As a result, a highly homologous sequence was found. Primers 1650Nde (SEQ ID NO:2) and 1650Bam (SEQ ID NO:3) were synthesized on the basis of the homologous sequence.

A PCR was carried out in a volume of 100 μl using 200 ng of the Pyrococcus furiosus DNA obtained in (1) above as a template, and 20 pmol of 1650Nde and 20 pmol of 1650Bam as primers. TaKaRa Ex Taq (Takara Shuzo) was used as a DNA polymerase for the PCR according to the attached protocol. The PCR was carried out as follows: 30 cycles of 94°C for 30 seconds, 55°C for 30 seconds and 72°C for 1 minute. An amplified DNA fragment of about 0.7 kb was digested with NdeI and BamhI (both from Takara Shuzo). The resulting DNA fragment was inserted between the NdeI site and the BamHI site in a plasmid vector pET3a (Novagen) to make a plasmid pPFU220.

(3) Determination of nucleotide sequence of DNA fragment containing RNase HII gene

The nucleotide sequence of the DNA fragment inserted into pPFU220 obtained in (2) above was determined according to a dideoxy method. Analysis of the determined nucleotide sequence revealed the existence of an open reading frame presumably encoding RNase HII. The nucleotide sequence of the open reading frame is shown in SEQ ID NO:4. The amino acid sequence of RNase HII deduced from the nucleotide sequence is shown in SEQ ID NO:5.

Escherichia coli JM109 transformed with the plasmid pPFU220 is designated and indicated as Escherichia coli JM109/pPFU220, and deposited on September 5, 2000 (date of original deposit) at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM P-18020 and transmitted to international depositary under accession no. FERM BP-7654 on July 9, 2001.

(4) Preparation of purified RNase HII preparation

Escherichia coli HMS174 (DE3) (Novagen) was transformed with pPFU220 obtained in (2) above. The resulting Escherichia coli HMS174(DE3) harboring pPFU220 was inoculated into 2 L of LB medium containing 100 μg/ml of ampicillin and cultured with shaking at 37°C for 16 hours. After cultivation, cells collected by centrifugation were suspended in 66.0 ml of a sonication buffer [50 mM tris-HCl (pH 8.0), 1 mM EDTA, 2 mM phenylmethanesulfonyl fluoride] and sonicated. A supernatant obtained by centrifuging the sonicated suspension at 12000 rpm for 10 minutes was heated at 60°C for 15 minutes. It was then centrifuged at 12000 rpm for 10 minutes again to collect a supernatant. Thus, 61.5 ml of a heated supernatant was obtained.

The heated supernatant was subjected to RESOURSE Q column (Amersham Pharmacia Biotech) equilibrated with Buffer A [50 mM tris-HCl (pH 8.0), 1 mM EDTA] and chromatographed using FPLC system (Amersham Pharmacia Biotech). As a result, RNase HII flowed through the RESOURSE Q column. 60.0 ml of the flow-through RNase HII fraction was subjected to RESOURSE S column (Amersham Pharmacia Biotech) equilibrated with Buffer A and eluted with a linear gradient of 0 to 500 mM NaCl using FPLC system. A fraction containing RNase HII eluted with about 150 mM NaCl was obtained. 2.0 ml of the RNase HII fraction was concentrated by ultrafiltration using Centricon-10 (Amicon). 250 μl of the concentrate was subjected to Superdex 200 gel filtration column (Amersham Pharmacia Biotech) equilibrated with 50 mM tris-HCl (pH 8.0) containing 100 mM NaCl and 0.1 mM EDTA and eluted with the same buffer. As a result, RNase HII was eluted at a position corresponding to a molecular weight of 17 kilodalton. This molecular weight corresponds to that of the RNase HII in the monomeric form. The thus eluted RNase HII was used as Pfu RNase HII preparation.

The RNase H activity was measured as described in Referential Example 3. As a result, an RNase H activity was observed for the Pfu RNase HII preparation.

Referential Example 2: Cloning of RNase HII gene from Pyrococcus horikoshii

(1) Preparation of genomic DNA from Pyrococcus horikoshii

2 L of a medium containing 1% Tryptone (Difco Laboratories), 0.5% yeast extract (Difco Laboratories), 1% soluble starch (Nacalai Tesque), 3.5% Jamarin S Solid (Jamarin Laboratory), 0.5% Jamarin S Liquid (Jamarin Laboratory), 0.003% $MgSO_4$, 0.001% NaCl, 0.0001% $FeSO_4 \cdot 7H_2O$, 0.0001% $CoSO_4$, 0.0001% $CaCl_2 \cdot 7H_2O$, 0.0001%

$ZnSO_4$, 0.1 ppm $CuSO_4 \cdot 5H_2O$, 0.1 ppm $KAl(SO_4)_2$, 0.1 ppm $H_3BO_4$, 0.1 ppm $Na_2MoO_4 \cdot 2H_2O$ and 0.25 ppm $NiCl_2 \cdot 6H_2O$ was placed in a 2-L medium bottle, sterilized at 120°C for 20 minutes, bubbled with nitrogen gas to remove dissolved oxygen, then Pyrococcus horikoshii OT3 (purchased from the Institute of Physical and Chemical Research (RIKEN); JCM9974) was inoculated into the medium and cultured at 95°C for 16 hours without shaking. After cultivation, cells were collected by centrifugation.

The cells were then suspended in 4 ml of 25% sucrose, 50 mM tris-HCl (pH 8.0). 0.4 ml of 10 mg/ml lysozyme chloride (Nacalai Tesque) in water was added thereto. The mixture was reacted at 20°C for 1 hour. After reaction, 24 ml of a mixture containing 150 mM NaCl, 1 mM EDTA and 20 mM tris-HCl (pH 8.0), 0.2 ml of 20 mg/ml proteinase K (Takara Shuzo) and 2 ml of 10% aqueous solution of sodium lauryl sulfate were added to the reaction mixture. The mixture was incubated at 37°C for 1 hour.

After reaction, the mixture was subjected to phenol-chloroform extraction followed by ethanol precipitation to prepare about 1 mg of genomic DNA.

(2) Cloning of RNase HII gene

The entire genomic sequence of the Pyrococcus horikoshii has been published [DNA Research, 5:55-76 (1998)]. The existence of one gene encoding a homologue of RNase HII (PH1650) was known (SEQ ID NO:1, the home page of National Institute of Technology and Evaluation: http://www.nite.go.jp/).

Primers PhoNde (SEQ ID NO:2) and PhoBam (SEQ ID NO:3) were synthesized on the basis of the sequence of the PH1650 gene (SEQ ID NO:51).

A PCR was carried out using 100 ng of the Pyrococcus horikoshii DNA prepared in Referential Example 2-(1) as a template, and 20 pmol each of PhoNde and PhoBam as primers in a volume of 100 μl. TaKaRa Ex Taq (Takara Shuzo) was used as a DNA polymerase for the PCR according to the attached protocol. The PCR was carried out as follows: 40 cycles of 94°C for 30 seconds, 55°C for 30 seconds and 72°C for 1 minute. An amplified DNA fragment of about 0.7 kb was digested with NdeI and BamHI (both from Takara Shuzo). Then, a plasmid pPHO238 was constructed by incorporating the resulting DNA fragment between NdeI and BamHI sites in a plasmid vector pET3a (Novagen).

(3) Determination of nucleotide sequence of DNA fragment containing RNase HII gene

The nucleotide sequence of the DNA fragment inserted into pPHO238 obtained in Referential Example 2-(2) was determined according to a dideoxy method.

Analysis of the determined nucleotide sequence revealed an open reading frame presumably encoding RNase HII. The nucleotide sequence of the open reading frame is shown in SEQ ID NO: 51. The amino acid sequence of RNase HII deduced from the nucleotide sequence is shown in SEQ ID NO:52.

Escherichia coli JM109 transformed with the plasmid pPHO238 is designated and indicated as Escherichia coli JM109/pPHO238, and deposited on February 22, 2001 (date of original deposit) at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM P-18222 and transmitted to international depositary under accession no. FERM BP-7692 on August 2, 2001.

(4) Preparation of purified RNase HII preparation

Escherichia coli HMS174(DE3) (Novagen) was transformed with pPHO238 obtained in Referential Example 2-(2). The resulting Escherichia coli HMS174(DE3) harboring pPHO238 was inoculated into 1 L of LB medium containing 100 μg/ml of ampicillin and cultured with shaking at 37°C for 16 hours. After cultivation, cells collected by centrifugation were suspended in 34.3 ml of a sonication buffer [50 mM tris-HCl (pH 8.0), 1 mM EDTA, 2 mM phenylmethanesulfonyl fluoride] and sonicated. A supernatant obtained by centrifuging the sonicated suspension at 12000 rpm for 10 minutes was heated at 80°C for 15 minutes. It was then centrifuged at 12000 rpm for 10 minutes again to collect a supernatant. Thus, 33.5 ml of a heated supernatant was obtained.

The heated supernatant was subjected to RESOURSE Q column (Amersham Pharmacia Biotech) equilibrated with Buffer A [50 mM tris-HCl (pH 8.0), 1 mM EDTA] and chromatographed using FPLC system (Amersham Pharmacia Biotech). As a result, RNase HII flowed through the RESOURSE Q column.

35.0 ml of the flow-through RNase HII fraction was dialyzed against 2 L of Buffer B (50 mM tris-HCl (pH 7.0), 1 mM EDTA) for 2 hours. The dialysis was repeated two more times. 34.5 ml of the dialyzed enzyme solution was subjected to RESOURSE S column (Amersham Pharmacia Biotech) equilibrated with Buffer B and eluted with a linear gradient of 0 to 500 mM NaCl using FPLC system. A fraction containing RNase HII eluted with about 155 mM NaCl was obtained.

Buffer B was added to 4.0 ml of the fraction to make the NaCl concentration to 50 mM. The mixture was subjected to HiTrap-heparin column (Amersham Pharmacia Biotech) equilibrated with Buffer B containing 50 mM NaCl and eluted with a linear gradient of 50 to 550 mM NaCl using FPLC system. As a result, a fraction containing RNase HII eluted with about 160 mM NaCl was obtained.

6.9 ml of the RNase HII fraction was concentrated by ultrafiltration using Centricon-10 (Millipore). Two portions each separated from 250 μl of the concentrate were subjected to Superose 6 gel filtration column (Amersham Pharmacia Biotech) equilibrated with 50 mM tris-HCl (pH 7.0) containing 100 mM NaCl and 0.1 mM EDTA and eluted with the same buffer. As a result, RNase HII was eluted at a position corresponding to a molecular weight of 24.5 kilodalton. This molecular weight corresponds to that of the RNase HII in the monomeric form.

The RNase HII eluted as described above was used as Pho RNase HII preparation.

The enzymatic activity of the thus obtained Pho RNase HII preparation was measured as described in Referential Example 3. As a result, an RNase H activity was observed for the Pho RNase HII preparation.

Referential Example 3: Measurement of heat-resistant RNase H activity

1 mg of poly(rA) or poly(dT) (both from Amersham Pharmacia Biotech) was dissolved in 1 ml of 40 mM tris-HCl (pH 7.7) containing 1 mM EDTA to prepare a poly(rA) solution and a poly(dT) solution.

The poly(rA) solution (to a final concentration of 20 μg/ml) and the poly(dT) solution (to a final concentration of 30 μg/ml) were then added to 40 mM tris-HCl (pH 7.7) containing 4 mM $MgCl_2$, 1 mM DTT, 0.003% BSA and 4% glycerol. The mixture was reacted at 37°C for 10 minutes and then cooled to 4°C at prepare a poly(rA)-poly(dT) solution.

1 μl of an enzyme solution was added to 100 μl of the poly(rA)-poly(dT) solution. The mixture was reacted at 40°C for 10 minutes. 10 μl of 0.5 M EDTA was added thereto to terminate the reaction. Absorbance at 260 nm was then measured. As a control, 10 μl of 0.5 M EDTA was added to the reaction mixture, the resulting mixture was reacted at 40°C for 10 minutes, and the absorbance was then measured. A value (difference in absorbance) was obtained by subtracting the absorbance for the control from the absorbance for the reaction in the absence of EDTA. Thus, the concentration of nucleotide released from poly(rA)-poly(dT) hybrid by the enzymatic reaction was determined on the basis of the difference in absorbance. One unit of an RNase H was defined as an amount of enzyme that increases $A_{260}$ corresponding to release of 1 nmol of ribonucleotide in 10 minutes calculated according to the following equation. If a diluted enzyme solution is used, the value obtained using the following equation was corrected based on the dilution rate:

Unit = [Difference in Absorbance x Reaction

Volume (ml)] / 0.0152

Example 1

Conditions for denaturing a double-stranded chimeric oligonucleotide containing a ribonucleotide according to the present invention into single strands were examined. Chimeric oligonucleotides (40-mer to 43-mer) each having an amino group at the 5' terminus, ICAN(6)/M13-47 (SEQ ID NO:6), IACN (6)/R1/M13-47 (SEQ ID NO:7), ICAN (6)/R2/M13-47 (SEQ ID NO:8) and ICAN (6) /R3/M13-47 (SEQ ID NO:9), were synthesized. The chimeric oligonucleotides having nucleotide sequences of SEQ ID NOS:7, 8 and 9 have 1, 2 and 3 ribonucleotide(s), respectively, in the middle portion and have a common sequence of 16 nucleotides on the 5'-terminal side and a common sequence of 24 nucleotides on the 3'-terminal side. The oligonucleotide represented by SEQ ID NO:6 is a combination of these common sequences and does not contain a ribonucleotide. Hereinafter, the 5' sequence and the 3' sequence are referred to as the ICAN(6) sequence and M13-47 sequence, respectively. In addition to these oligonucleotides, a 40-mer oligonucleotide was synthesized using a DNA synthesizer (ABI) as a negative control. The oligonucleotide is modified with an amino group at the 5' terminus and has a sequence of SEQ ID NO:10 which is a part of the sequence or Escherichia coli succinate dehydrogenase gene (GenBank accession no. X00980).

Each of the chimeric oligonucleotide was dissolved at a final concentration of 0.1 mM in 40 mM carbonate buffer (pH 9.5) containing 0.2% sucrose monolaurate (Dojindo). For each sample, ten spots were spotted onto Mirus-E slide glass, which is a slide glass having an introduced activated carboxyl group prepared according to the method as described in WO 01/02538, using Affymetrix 417 Arrayer (Affymetrix). After spotting, the slide glass was washed in 0.2% SDS and in distilled water twice. The slide glass was treated under four types of denaturation conditions as follows:

1. control: the slide glass washed in distilled water was dried by low-speed centrifugation;
2. 0.2 N NaOH (20°C): the slide glass was treated in 0.2 N NaOH at 20°C for 5 minutes, washed in distilled water for 5 minutes, and then dried;

3. 0.5 N NaOH (20°C): the slide glass was treated in 0.5 N NaOH at 20°C for 5 minutes, washed in distilled water for 5 minutes, and then dried;
4. 0.2 N NaOH (65°C): the slide glass was treated in 0.2 N NaOH at 65°C for 30 minutes, washed in distilled water for 5 minutes, and then dried;
5. 0.5 N NaOH (65°C): the slide glass was treated in 0.5 N NaOH at 65°C for 30 minutes, washed in distilled water for 5 minutes, and then dried.

An oligonucleotide probe 1 (SEQ ID NO:11; labeled with Cy3 (Amersham Pharmacia Biotech) at the 5' terminus) and an oligonucleotide probe 2 (SEQ ID NO:12; labeled with Cy5 (Amersham Pharmacia Biotech) at the 5' terminus) as probes for detecting oligonucleotide sequences on the arrays were synthesized using a DNA synthesizer. The oligonucleotide probes 1 and 2 are complementary to the ICAN(6) sequence and the M13-47 sequence, respectively.

Hybridization was carried out using a prehybridization solution and a hybridization solution which were prepared according to the instructions attached to a commercially available product IntelliGene (Takara Shuzo). The array was subjected to prehybridization at 20°C for 2 hours. A hybridization solution containing one of the labeled probes at a concentration of 10 pmol/ul was prepared, heated at 95°C for 2 minutes, and added dropwise onto the DNA array. The array was covered with a cover glass and the sides were sealed with paper glue (Kokuyo). After incubation at 42°C for 13 hours, the cover glass was removed. The array was washed in a solution containing 2 x SSC and 0.2% SDS at 45°C for 10 minutes, rinsed with 0.05 x SSC at 20°C, and dried by centrifugation. Fluorescence images for Cy3 (excitation wavelength: 532 nm; emission wavelength: 570 nm) and Cy5 (excitation wavelength: 635 nm; emission wavelength: 670 nm) were obtained using Affymetrix 418 Array Scanner (Affymetrix). Signal intensities for the respective spots were quantified using an expression data analysis software ImaGene 4.0 (Biodiscovery). Then, a ratio of a signal from the M13-47 sequence to a signal from the ICAN(6) sequence, i.e., a ratio of a value for a fluorescence signal from Cy5 to a value for a fluorescence signal from Cy3 was determined. The results are shown in Table 1. Table 1 shows an average ratio for ten spots of each oligonucleotide on each slide glass defining the ratio for the ICAN(6)/M13-47 oligonucleotide (SEQ ID NO:6) as 100%. Since no fluorescence signal was detected from a spot as a negative control, it was confirmed that the signals from the spots of the oligonucleotides were due to sequence specificities.

Table 1

| | Number of ribonucleotides included | | | |
|---|---|---|---|---|
| | 0 | 1 | 2 | 3 |
| No treatment | 100% | 102% | 75% | 77% |
| 0.2 N NaOH, 5 minutes (20°C) | 100% | 99% | 75% | 75% |
| 0.5 N NaOH, 5 minutes (20°C) | 100% | 101% | 70% | 70% |
| 0.2 N NaOH, 30 minutes (65°C) | 100% | 5% | 3% | 3% |
| 0.5 N NaOH, 30 minutes (65°C) | 100% | 4% | 3% | 3% |

As shown in Table 1, the decrease in the ratio due to the increase in the number of ribonucleotides observed for the slide glass with treatment in 0.2 N NaOH at 20°C for 5 minutes or in 0.5 N NaOH at 20°C for 5 minutes was equivalent to that observed for the slide glass with no treatment. Based on these results, it was confirmed that treatment at a temperature of 10°C to 40°C in a solution containing an alkali at a concentration of 0.1 N to 2.0 N for a period of 5 minutes to 30 minutes results in almost no cleavage of a chimeric oligonucleotide on a solid phase at a ribonucleotide portion. In particular, it was confirmed that alkali denaturation in 0.2 N NaOH at 20°C for 5 minutes or in 0.5 N NaOH at 20°C for 5 minutes is preferable.

Example 2

Examination was carried out for an array having an immobilized practical double-stranded nucleic acid amplified by the ICAN method using the denaturation conditions found in Example 1.

Amplification products corresponding to two open reading frames (ORFs) from Escherichia coli K-12 were prepared in this Example. The ORFs, ORF ID No. 131#6 and ORF ID NO. 214#1, are published in GenoBase (http://ecoli.aist-nara.ac.jp/) on the Internet. Accordingly, information on the nucleotide sequences of the ORFs and the expression products thereof was obtained from the database.

Conditions used for amplifying a nucleic acid by the ICAN method were as follows. Briefly, amplified fragments for 131#6 and 214#1 were prepared by the PCR method using a genomic DNA from Escherichia coli K-12 strain W3110 as a template and a pair of primers having sequences of SEQ ID NOS:13 and 14 or SEQ ID NOS:15 and 16. The amplified fragment (300 bp) was cloned into a vector pUC19 (Takara Shuzo) and PCR-amplified using primers having nucleotide sequences of SEQ ID NOS:17 and 18. 20 μl of a mixture containing 100 pmol each of primers having nucleotide sequences of SEQ ID NOS:19 and 20, 100 ng of the PCR amplification product as a template and propylenediamine at a final concentration of 0.01% was then prepared. The mixture was incubated at 98°C for 2 minutes and then placed on ice. A reaction mixture (final volume of 100 μl) containing the mixture and, at final concentration, 20 mM HEPES-potassium hydroxide buffer (pH 7.8), 100 mM potassium acetate, 0.01% bovine serum albumin (BSA), 1% dimethyl sulfoxide, 4 mM magnesium acetate, 0.5 mM each of dNTPs, 11 U of BcaBEST DNA polymerase (Takara Shuzo) and 60 U of Escherichia coli type I RNase H (Takara Shuzo) was prepared. The reaction mixture was mixed to homogeneity and incubated at 60°C for 60 minutes. Primers having the sequence of SEQ ID NO:19 with or without modification with an amino group at the 5' terminus were used. As a result, a double-stranded nucleic acid containing an amino group in the primer (referred to as an ICAN product 1) and a double-stranded nucleic acid containing no amino group (referred to as an ICAN product 2) were obtained for each ORF.

A double-stranded nucleic acid was amplified using the PCR method. Specifically, fragments for 131#6 and 214#1 were PCR-amplified using a genomic DNA from Escherichia coli K-12 strain W3110 as a template and a pair of primers having nucleotide sequences of SEQ ID NOS:13 and 14 or SEQ ID NOS:15 and 16. The amplified fragment was cloned into a vector pUC19. PCR amplification was carried out using the clone as a template and primers having nucleotide sequences of SEQ ID NOS:21 and 22. Primers having the nucleotide sequence of SEQ ID NO:21 with or without modification with an amino group at the 5' terminus were used. As a result, a double-stranded nucleic acid containing an amino group in the primer (referred to as a PCR product 1) and a double-stranded nucleic acid containing no amino group (referred to as a PCR product 2) were obtained for each ORF.

The above-mentioned four types of double-stranded amplified nucleic acids were purified using QIAquick PCR purification Kit (Qiagen) according to the protocol attached thereto. The purified nucleic acid was dissolved at a final concentration of 0.3 μg/μl in 40 mM carbonate buffer (pH 9.5) containing 0.1% sucrose monolaurate. For each sample, ten spots were spotted onto Mirus-E slide glass, which is a slide glass having an introduced activated carboxyl group, using Affymetrix 417 Arrayer. After spotting, the slide glass was washed in 0.2% SDS and in distilled water twice. After drying, the slide glass was treated under four types of conditions as follows:

1. no treatment;
2. the slide glass was treated in 0.2 N NaOH at 20°C for 5 minutes, washed in distilled water for 5 minutes, and then dried;
3. the slide glass was treated in 0.5 N NaOH at 20°C for 5 minutes, washed in distilled water for 5 minutes, and then dried;
4. the slide glass was treated in 0.2 N NaOH at 65°C for 30 minutes, washed in distilled water for 5 minutes, and then dried.

Prehybridization, hybridization, washing and drying were carried out according to a protocol as described in instructions attached to IntelliGene using an oligonucleotide as a probe. The oligonucleotide was labeled with Cy5 at the 5' terminus and had a sequence of SEQ ID NO:23 which is complementary to a sequence of the pUC19 portion commonly contained in all the amplification products. Scanning was then carried out at the wavelength for Cy5 to obtain a fluorescence image. Signal intensities for the respective spots and the intensity for the surrounding background were quantified using ImaGene 4.0. Results of the signal intensities for the respective spots are shown in Table 2. Each value in the table represents a mean value for ten spots.

Table 2

| ORF ID | Type of treatment for single strand conversion | ICAN product 1 | ICAN product 2 | PCR product 1 | PCR product 2 |
|---|---|---|---|---|---|
| 131#6 | No treatment | 2808 | 2531 | 5875 | 965 |
| | 0.2 N NaOH 20°C 5 minutes | 4792 | 2791 | 9067 | 2135 |
| | 0.2 N NaOH 65°C 30 minutes | 7245 | 6080 | 50282 | 4457 |
| | Boiling water 3 minutes | 19105 | 13310 | 44884 | 8986 |
| 140#9 | No treatment | 1616 | 1550 | 3867 | 1546 |
| | 0.2 N NaOH 20°C 5 minutes | 3243 | 1817 | 12130 | 3784 |
| | 0.2 N NaOH 65°C 30 minutes | 5878 | 4582 | 46289 | 4592 |
| | Boiling water 3 minutes | 13911 | 9267 | 40264 | 10939 |

Firstly, signal intensities were compared between the ICAN products and the PCR products. As shown in Table 2, the signal intensity observed for the ICAN product 1 was substantially equivalent to that observed for the PCR product 1 in case of the slide glass with no treatment for single strand conversion, alkali treatment in 0.2 N NaOH at 20°C for 5 minutes or heat treatment. Based on these results, it was confirmed that a double-stranded ICAN product, or a partially single-stranded ICAN product, modified with an amino group can be preferably used as a nucleic acid to be immobilized upon preparation of a covalent bond-type microarray. On the other hand, in case of the slide glass with treatment in 0.2 N NaOH at 65°C for 30 minutes, the signal intensity observed for the ICAN product 1 was substantially lower than that observed for the PCR product 1. The lowering was supposed to be due to degradation of the ribonucleotide portion in the primer contained in the ICAN product under the denaturation conditions as demonstrated in Example 1.

Secondly, signal intensities were compared between the slide glass with no treatment and the slide glass with treatment for single strand conversion with respect to the ICAN product 1 and the PCR product 1. As shown in Table 2, the signal intensities observed for the slide glasses with alkali treatment in 0.2 N NaOH at 20°C for 5 minutes and heat treatment were higher than the signal intensity observed for the slide glass with no treatment. Based on these results, it was confirmed that an efficiency of capturing a probe onto a solid phase (i.e., a hybridization efficiency) of a product in a single-stranded form is higher than that of a product in a double-stranded form whether the product is an ICAN product or a PCR product. Also, the signal intensities observed for the spots of the PCR product 1 and the ICAN product 1 on the slide glass with treatment in 0.2 N NaOH at 65°C for 30 minutes were higher than those on the slide glass with no treatment. It was supposed that this was because the decrease in nucleic acid amount due to detachment as a result of degradation of the ribonucleotide portion was compensated by the increase in hybridization efficiency due to conversion into single strands.

Example 3

Signal intensities observed upon hybridization were compared between a microarray composed of a single-stranded or double-stranded ICAN product immobilized onto a solid phase via covalent bond as described in Example 2 and a conventional microarray having a double-stranded PCR product immobilized onto a solid phase via an electrostatic bond.

A conventional microarray was prepared as follows. Briefly, each of the four types of double-stranded nucleic acids for the respective ORFs amplified and purified in Example 2 was dissolved at a final concentration of 0.3 μg/μl in 40 mM carbonate buffer (pH 9.5) containing 0.05% sucrose monolaurate. For each sample, ten spots were spotted onto MAS-coated slide glass (Matsunami Glass), which is a slide glass having an introduced activated amino group, using Affymetrix 417 Arrayer. After spotting, the slide glass was incubated at 37°C in a humidified incubator for 1 hour, and then subjected to UV irradiation at 60 $mJ/cm^2$. The slide glass was washed in a 0.2% SDS solution followed by three times in distilled water. The free amino group was blocked by soaking the slide glass in a blocking solution for 20 minutes. The blocking solution was prepared immediately before use by dissolving 2.75 g of succinic anhydride in 160 ml of N-methyl-2-pyrrolidone, and adding thereto 15 ml of 1 M borate buffer (pH 8.0). Finally, the slide glass was washed twice in distilled water, centrifuged to remove water, and stored in a desiccator.

The conventional microarray prepared as described above was subjected to prehybridization, hybridization, washing and drying according to a protocol as described in instructions attached to IntelliGene using an oligonucleotide as a probe. The oligonucleotide was labeled with Cy5 at the 5' terminus and had a sequence of SEQ ID NO:23 which is complementary to a sequence of the pUC19 portion commonly contained in the respective amplification products. Scanning of the slide glass was then carried out at the wavelength for Cy5 to obtain a fluorescence image. Signal intensities for the respective spots and the intensity for the surrounding background were quantified using ImaGene 4.0. A ratio of a signal intensity to a background intensity (hereinafter referred to as an S/N ratio) was calculated. The calculation was also conducted for the results as shown in Table 2 in Example 2 in order to compare the MAS-coated slide glass with the Mirus-E slide glass. The results are shown in Table 3.

Table 3

| | | ORF ID | ICAN product 1 | ICAN product 2 | PCR product 1 | PCR product 2 |
|---|---|---|---|---|---|---|
| MAS-coated slide glass | | 131#6 | 13.0 | 13.7 | 8.7 | 7.6 |
| | | 214#1 | 6.8 | 7.7 | 9.7 | 8.7 |
| Mirus-E slide glass | No treatment | 131#6 | 3.1 | 3.0 | 5.6 | 1.8 |
| | | 214#1 | 2.4 | 2.2 | 4.0 | 2.2 |

Table 3 (continued)

| | | ORF ID | ICAN product 1 | ICAN product 2 | PCR product 1 | PCR product 2 |
|---|---|---|---|---|---|---|
| | 0.2 N NaOH | 131#6 | 4.8 | 3.4 | 8.5 | 2.6 |
| | 20°C 5 min | 214#1 | 3.4 | 2.5 | 10.6 | 4.0 |
| | 0.2 N NaOH | 131#6 | 6.8 | 5.6 | 37.6 | 4.3 |
| | 65°C 30 min | 214#1 | 5.5 | 4.4 | 33.9 | 4.4 |
| | Heat treatment | 131#6 | 15.6 | 10.9 | 32.8 | 7.8 |
| | | 214#1 | 11.8 | 8.3 | 28.9 | 8.6 |

As shown in Table 3, the signal intensity observed for the spot of the ICAN product 1 on the slide glass with no treatment or with each of the three types of treatments for single strand conversion as described in Example 2 was only slightly lower than that observed for the spot of the PCR product 1 on the conventional microarray. Based on these results, it was confirmed that a material having an immobilized nucleic acid in which a single-stranded or double-stranded ICAN product is immobilized via a covalent bond can be preferably used as a microarray like a conventional material in which a double-stranded PCR product is immobilized via an electrostatic bond.

Example 4

Properties of ICAN products obtained using Pfu RNase HII and Pho RNase HII were examined. ICAM amplification was carried out as follows. Briefly, a PCR was carried out using a plasmid pUC19 as a template and primers "upper150" and "lower" having nucleotide sequences of SEQ ID NOS:49 and 50 to obtain an about 150-bp amplified fragment. The amplified fragment was purified and cloned into the plasmid pUC19 to obtain a plasmid DNA (designated as pUC19-150) which was used as a template in an ICAN reaction.

DNA fragments were PCR-amplified using a combination of primers ICAN(6)-M4 (SEQ ID NO:24) and ICAN (6)-RV (SEQ ID NO:25) or ICANVFN3-M4 (SEQ ID NO:26) and ICANVFN3-RVM (SEQ ID NO:27) as primers. An ICAN reaction was carried out using one of the above-mentioned DNA fragments as a template and a chimeric oligonucleotide primer ICAN(6) (SEQ ID NO:28) or VFN3 (SEQ ID NO:29) as a primer as follows.

(1) 10 μl of a mixture was prepared by adding 100 pmol of ICAN(6) primer, 0.01% propylenediamine, and 1 μl of the PCR reaction mixture obtained using the combination of primers ICAN(6)-M4 and ICAN(6)-RV. The mixture was incubated at 98°C for 2 minutes and 60°C for 1 minute and using Thermal Cycler Personal (Takara Shuzo) then placed on ice. A mixture (final volume of 50 μl) was prepared by adding a mixture containing, at final concentrations, 32 mM HEPES-potassium hydroxide buffer (pH 7.8), 100 mM potassium acetate, 1% dimethyl sulfoxide, 0.01% bovine serum albumin, 4 mM magnesium acetate, 500 μM each of dNTPs, and a combination of 0.0156 μg of Pfu RNase HII and 2 U of BcaBEST DNA polymerase or a combination of 5 U of Pho RNase HII and 8 U of BcaBEST DNA polymerase to the above-mentioned mixture. The reaction mixture was placed in a thermal cycler which had been set at 60°C and incubated for 60 minutes.

(2) 10 μl of a mixture was prepared by adding 100 pmol of the primer VFN3, 0.01% propylenediamine, and 1 μl of the PCR reaction mixture obtained using the combination of primers ICANVFN3-M4 and ICANVFN3-RVM. The mixture was incubated at 98°C for 2 minutes and 55°C for 1 minute and using Thermal Cycler Personal (Takara Shuzo) then placed on ice. A mixture (final volume of 50 μl) was prepared by adding a mixture containing, at final concentrations, 32 mM HEPES-potassium hydroxide buffer (pH 7.8), 100 mM potassium acetate, 1% dimethyl sulfoxide, 0.01% bovine serum albumin, 4 mM magnesium acetate, 500 μM each of dNTPs, and a combination of 0.0312 μg of Pfu RNase HII and 4 U of BcaBEST DNA polymerase or a combination of 5 U of Pho RNase HII and 8 U of BcaBEST DNA polymerase to the above-mentioned mixture. The reaction mixture was placed in a thermal cycler which had been set at 55°C and incubated for 60 minutes.

(3) In addition, reactions were carried out using Escherichia coli RNase H as controls. Specifically, reaction mixtures having compositions similar to those as described in (1) and (2) above were prepared and used except that 30 U of Escherichia coli RNase H and 5.5 U of BcaBEST DNA polymerase were used.

After reaction, 1.5 μl each of the reaction mixtures was subjected to electrophoresis on 3% NuSieve 3:1 Agarose (Takara Shuzo) to confirm the amplification product. The expected size of the amplification product was about 300 bp. After confirming the amplification product, the reaction mixture was subjected to purification using Microcon-100 spin column (Takara Shuzo) and the concentration in the purified solution was determined using an absorbance photometer.

1 μg of the thus obtained purified ICAN product was placed in a tube, and an NaOH solution (to a final concentration of 1 N) was added thereto for alkali treatment at 60°C for 1 hour. After treatment, 6 μl each of the mixtures was subjected to electrophoresis on 4% denaturing acrylamide gel.

Three bands for ICAN products are observed upon electrophoresis, i.e., one having primers at both ends, one having a primer at one end and one having no primer. By comparison with the product without alkali treatment, it was confirmed that the ICAN product having primers at both ends obtained using Escherichia coli RNase H and the primer ICAN(6) or VFN3 disappeared upon alkali treatment. On the other hand, the ICAN product obtained using Pfu RNase HII or Pho RNase HII and the primer ICAN(6) or VFN3 was not altered upon alkali treatment as compared with the product with no treatment.

As described above, it was confirmed that an ICAN product obtained using Escherichia coli RNase H contained an RNA portion derived from a chimeric primer used for ICAN, whereas an ICAN product obtained using Pfu RNase HII or Pho RNase HII did not contain such an RNA portion.

Example 5

(1) Preparation of template for ICAN reaction by PCR

A double-stranded cDNA was prepared using mouse brain polyA+ RNA (OriGene) and a cDNA synthesis kit (Takara Shuzo). A PCR fragment amplified using a combination of primers selected from those having nucleotide sequences of SEQ ID NOS:30 to 43 and the double-stranded cDNA as a template was subjected to TA cloning into pT7 Blue T-vector (Takara Shuzo) to obtain a plasmid clone. 50 μl of a mixture containing 1 μl of the plasmid clone (1 ng), 10 pmol each of primers MCS-F and MCS-R having nucleotide sequences of SEQ ID NOS:44 and 45, 1.25 U of Ex Taq (Takara Shuzo), 5 μl of 10 x Ex Taq buffer (Takara Shuzo) and 0.2 mM each of dNTPs was subjected to a reaction using TaKaRa PCR Thermal Cycler Personal (Takara Shuzo) as follows: 94°C for 2 minutes; 30 cycles of 94°C for 30 seconds, 55°C for 30 seconds and 72°C for 1 minute. The thus obtained amplified DNA fragment was used as a template in an ICAN reaction.

(2) Amplification of DNA fragment by ICAN method using PCR product as template

An ICAN reaction was carried out using Aminoallyl dUTP (Sigma) in order to incorporate an amino group into an ICAN amplification product. The ratio of amino group to be incorporated into an ICAN amplification product was examined by carrying out ICAN reactions with varying ratios of the amount of dTTP to the amount of Aminoallyl dUTP as follows: 10:0, 9:1, 8:2, 7:3, 6:4. The reaction was carried out as follows.

10 μl of a mixture containing 1 μl of the PCR reaction mixture prepared in (1) above, 50 pmol each of primers MF2N3(24) and MR1N3(24) having nucleotide sequences of SEQ ID NOS:47 and 48 and 2 μl of a 0.05% propylenediamine aqueous solution was prepared. The mixture was heated at 98°C for 2 minutes and 65°C for 30 seconds using TaKaRa PCR Thermal Cycler Personal, and then rapidly cooled on ice for annealing the primers to the template. 40 μl of a reaction mixture containing 0.625 mM each of dATP, dCTP and dGTP, 0.625 mM of a dTTP + Aminoallyl dUTP mixture, 32 mM Hepes-potassium hydroxide buffer (pH 7.8), 5.0 mM magnesium acetate, 0.6 U of Escherichia coli RNase H (Takara Shuzo), 2.75 U of BcaBEST DNA polymerase was added to the heated mixture. The resulting mixture was incubated at 65°C for 1 hour using a thermal cycler.

50 μl of isopropanol and 5 μl of a 3 M sodium acetate solution (pH5.2) were added to 50 μl of the reaction mixture. After allowing to stand at -80°C for 20 minutes, the mixture was centrifuged and a supernatant was removed. Subsequently, 200 μl of a 70% ethanol solution was added thereto, the mixture was centrifuged, a supernatant was removed, and the precipitate was air-dried. The thus obtained DNA was re-dissolved in water and the amount of the product was determined by measuring $OD_{260/280}$.

(3) Confirmation of incorporation of Aminoallyl dUTP into ICAN amplification product

Incorporation of an amino group into an ICAN product was confirmed by fluorescence-labeling the amino group in the ICAN product using 5-carboxyfluorescein succinimidil ester (Molecular Probes). A portion of the DNA solution was diluted to a concentration of 2 μg/50 μl. 20 μl of 1 M sodium carbonate buffer (pH 9.0) was added thereto followed by 4 μl of a solution containing FITC (Nacalai Tesque) at a concentration of 10 mM in N,N-dimethylformamide. The mixture was reacted at 20°C for 16 hours. After removing excess FITC using a commercially available spin column, 10 μl of the mixture was applied to 2.0% agarose gel and electrophoresed. After electrophoresis, the fluorescent dye was observed using FM-BIO, and the ICAN-amplified fragment was observed after staining with EtBr. As a result, it was confirmed that an amino group could be incorporated into an ICAN amplification product by carrying out ICAN using Aminoallyl dUTP.

(4) Application of ICAN product having incorporated Aminoallyl dUTP to DNA microarray

ICAN products having incorporated amino groups were prepared for six mouse genes (ribosomal protein S18, transferrin receptor, stromal cell derived factor 4, cytoplasmic beta-actin, ornithine decarboxylase, tyrosine 3-monooxygenase) in a manner similar to that as described above for HPRT gene. Each solution corresponding to 9 μg of the DNA was placed in a well of a 96-well assay block (Advanced BIOTECHNOLOGIES) and lyophilized. The ICAN products were used to prepare a DNA microarray as follows. Briefly, the ICAN product was dissolved at a final concentration of 0.3 μg/μl in 40 mM carbonate buffer (pH 9.5) containing 0.1% sucrose monolaurate. For each sample, two spots were spotted onto Mirus-E slide glass, which is a slide glass having an introduced activated carboxyl group, using Affymetrix 417 Arrayer. After spotting, the slide glass was washed in 0.2% SDS and in distilled water twice. The slide glass was treated under four types of conditions as follows:

treatment 1. control: the slide glass washed in distilled water was dried by low-speed centrifugation;
treatment 2. 0.2 N NaOH (20°C): the slide glass was treated in 0.2 N NaOH at 20°C for 5 minutes, washed twice in distilled water for 5 minutes, and then dried;
treatment 3. 0.2 N NaOH (65°C): the slide glass was treated in 0.2 N NaOH at 65°C for 30 minutes, washed twice in distilled water for 5 minutes, and then dried;
treatment 4. the slide glass was heated (3 minutes in boiling water), rapidly cooled in ice-cold 99.5% ethanol, and then dried.

After the denaturation, the DNA microarray of ICAN products was subjected to prehybridization, hybridization, washing and drying according to a protocol as described in instructions attached to IntelliGene using an oligonucleotide labeled with Cy5 at the 5' terminus (SEQ ID NO:46) as a probe. The oligonucleotide probe had a sequence complementary to a vector portion commonly contained in strands of the ICAN amplification products extended from the ICAN primer MF2N3(24). Scanning of the slide glass was then carried out at the wavelength for Cy5 to obtain a fluorescence image. Signal intensities for the respective spots were quantified using ImaGene 4.0. The results are shown in Tables 4 to 10. Tables 4 to 10 show ratios of mixed dTTP and Aminoallyl dUTP, values for fluorescence signals from DNA microarrays measured after the respective treatments ("Signal" in tables) and NH2 ratios ("NH2" in tables; values for fluorescence signals measured using the mixtures with the respective ratios defining the fluorescence signal measured using the mixture without Aminoallyl dUTP as 1).

Table 4

| Ribosomal protein S18 | | | | | | | | |
|---|---|---|---|---|---|---|---|---|
| dTTP: Aminoallyl dUTP | Treatment 1 | | Treatment 2 | | Treatment 3 | | Treatment 4 | |
| | Signal | NH2 | Signal | NH2 | Signal | NH2 | Signal | NH2 |
| 10:0 | 943.15 | 1.0 | 1284.83 | 1.0 | 2838.77 | 1.0 | 3425.97 | 1.0 |
| 9:1 | 965.55 | 1.0 | 2308.20 | 1.8 | 6521.76 | 2.3 | 4908.82 | 1.4 |
| 8:2 | 1809.03 | 1.9 | 3952.96 | 3.1 | 12849.96 | 4.5 | 8059.78 | 2.4 |
| 7:3 | 2296.15 | 2.4 | 4963.44 | 3.9 | 15626.19 | 5.5 | 9520.15 | 2.8 |
| 6:4 | 2922.25 | 3.1 | 7811.34 | 6.1 | 24300.50 | 8.6 | 12792.03 | 3.7 |

Table 5

| Transferrin receptor | | | | | | | | |
|---|---|---|---|---|---|---|---|---|
| dTTP: Aminoallyl dUTP | Treatment 1 | | Treatment 2 | | Treatment 3 | | Treatment 4 | |
| | Signal | NH2 | Signal | NH2 | Signal | NH2 | Signal | NH2 |
| 10:0 | 946.80 | 1.0 | 1198.71 | 1.0 | 1844.43 | 1.0 | 3113.70 | 1.0 |
| 9:1 | 1025.29 | 1.1 | 2363.24 | 2.0 | 4491.01 | 2.4 | 3717.36 | 1.2 |
| 8:2 | 1030.52 | 1.1 | 2104.35 | 1.8 | 6834.68 | 3.7 | 4792.72 | 1.5 |
| 7:3 | 1295.40 | 1.4 | 2160.94 | 1.8 | 8839.25 | 4.8 | 5520.32 | 1.8 |

Table 5 (continued)

| Transferrin receptor | | | | | | | | |
|---|---|---|---|---|---|---|---|---|
| dTTP: Aminoallyl dUTP | Treatment 1 | | Treatment 2 | | Treatment 3 | | Treatment 4 | |
| | Signal | NH2 | Signal | NH2 | Signal | NH2 | Signal | NH2 |
| 6:4 | 1974.26 | 2.1 | 3143.59 | 2.6 | 9409.99 | 5.1 | 5041.05 | 1.6 |

Table 6

| Stromal cell derived factor 4 | | | | | | | | |
|---|---|---|---|---|---|---|---|---|
| dTTP: Aminoallyl dUTP | Treatment 1 | | Treatment 2 | | Treatment 3 | | Treatment 4 | |
| | Signal | NH2 | Signal | NH2 | Signal | NH2 | Signal | NH2 |
| 10:0 | 643.94 | 1.0 | 967.35 | 1.0 | 2325.25 | 1.0 | 2985.70 | 1.0 |
| 9:1 | 984.42 | 1.5 | 1436.37 | 1.5 | 4684.63 | 2.0 | 4305.73 | 1.4 |
| 8:2 | 1018.52 | 1.6 | 1600.25 | 1.7 | 6080.83 | 2.6 | 4214.09 | 1.4 |
| 7:3 | 1562.79 | 2.4 | 2014.86 | 2.1 | 6921.77 | 3.0 | 5196.68 | 1.7 |
| 6:4 | 1285.41 | 2.0 | 2316.74 | 2.4 | 7752.27 | 3.3 | 3785.39 | 1.3 |

Table 7

| Cytoplasmic beta-actin | | | | | | | | |
|---|---|---|---|---|---|---|---|---|
| dTTP: Aminoallyl dUTP | Treatment 1 | | Treatment 2 | | Treatment 3 | | Treatment 4 | |
| | Signal | NH2 | Signal | NH2 | Signal | NH2 | Signal | NH2 |
| 10:0 | 548.80 | 1.0 | 953.13 | 1.0 | 1753.28 | 1.0 | 2479.78 | 1.0 |
| 9:1 | 917.48 | 1.7 | 1060.15 | 1.1 | 3772.38 | 2.2 | 2884.40 | 1.2 |
| 8:2 | 949.57 | 1.7 | 1231.43 | 1.3 | 4591.59 | 2.6 | 3197.53 | 1.3 |
| 7:3 | 1775.48 | 3.2 | 2125.71 | 2.2 | 6569.03 | 3.7 | 3912.09 | 1.6 |
| 6:4 | 1859.68 | 3.4 | 2512.72 | 2.6 | 9208.45 | 5.3 | 5410.43 | 2.2 |

Table 8

| Ornithine decarboxylase | | | | | | | | |
|---|---|---|---|---|---|---|---|---|
| dTTP: Aminoallyl dUTP | Treatment 1 | | Treatment 2 | | Treatment 3 | | Treatment 4 | |
| | Signal | NH2 | Signal | NH2 | Signal | NH2 | Signal | NH2 |
| 10:0 | 540.97 | 1.0 | 1482.14 | 1.0 | 3162.20 | 1.0 | 5068.03 | 1.0 |
| 9:1 | 648.09 | 1.2 | 1192.15 | 0.8 | 3452.62 | 1.1 | 3162.37 | 0.6 |
| 8:2 | 941.75 | 1.7 | 1132.60 | 0.8 | 3579.80 | 1.1 | 2561.54 | 0.5 |
| 7:3 | 1470.83 | 2.7 | 1676.83 | 1.1 | 4289.90 | 1.4 | 2814.37 | 0.6 |
| 6:4 | 1282.93 | 2.4 | 1784.22 | 1.2 | 4906.50 | 1.6 | 3226.55 | 0.6 |

Table 9

| Hypoxanthine guanine phophoribosyl transferase | | | | | | | | |
|---|---|---|---|---|---|---|---|---|
| dTTP: Aminoallyl dUTP | Treatment 1 | | Treatment 2 | | Treatment 3 | | Treatment 4 | |
| | Signal | NH2 | Signal | NH2 | Signal | NH2 | Signal | NH2 |
| 10:0 | 1574.17 | 1.0 | 1814.48 | 1.0 | 2935.35 | 1.0 | 4206.51 | 1.0 |
| 9:1 | 2939.24 | 1.9 | 3132.06 | 1.7 | 8093.26 | 2.8 | 7540.02 | 1.8 |
| 8:2 | 4335.98 | 2.8 | 3890.58 | 2.1 | 10695.84 | 3.6 | 7848.82 | 1.9 |

Table 9 (continued)

| Hypoxanthine guanine phophoribosyl transferase | | | | | | | | |
|---|---|---|---|---|---|---|---|---|
| dTTP: Aminoallyl dUTP | Treatment 1 | | Treatment 2 | | Treatment 3 | | Treatment 4 | |
| | Signal | NH2 | Signal | NH2 | Signal | NH2 | Signal | NH2 |
| 7:3 | 5391.38 | 3.4 | 6327.24 | 3.5 | 17589.95 | 6.0 | 10455.70 | 2.5 |
| 6:4 | 7263.77 | 4.6 | 8342.32 | 4.6 | 21607.64 | 7.4 | 12681.97 | 3.0 |

Table 10

| Tyrosine 3-monooxygenase | | | | | | | | |
|---|---|---|---|---|---|---|---|---|
| dTTP: Aminoallyl dUTP | Treatment 1 | | Treatment 2 | | Treatment 3 | | Treatment 4 | |
| | Signal | NH2 | Signal | NH2 | Signal | NH2 | Signal | NH2 |
| 10:0 | 1078.40 | 1.0 | 1606.78 | 1.0 | 2563.29 | 1.0 | 3861.78 | 1.0 |
| 9:1 | 1682.53 | 1.6 | 2040.56 | 1.3 | 5566.44 | 2.2 | 5844.36 | 1.5 |
| 8:2 | 2183.62 | 2.0 | 2494.04 | 1.6 | 7546.58 | 2.9 | 7332.19 | 1.9 |
| 7:3 | 2838.70 | 2.6 | 3267.69 | 2.0 | 8086.52 | 3.2 | 7099.06 | 1.8 |
| 6:4 | 3033.00 | 2.8 | 251.27 | 2.6 | 10162.59 | 4.0 | 8474.05 | 2.2 |

As shown in Tables 4 to 10, it was confirmed that a weak signal was generally observed in the absence of denaturation, indicating inefficient hybridization to a probe. However, the signal intensity observed for the product obtained by carrying out an ICAN reaction using Aminoallyl dUTP was higher than that observed for the product containing no Aminoallyl dUTP. The signal intensity was increased as the Aminoallyl dUTP content was increased. These results suggest that the presence of the incorporated amino group increased the efficiency of immobilization of an ICAN-amplified nucleic acid onto a slide glass.

The signal intensity observed with denaturation was higher than that without denaturation. Thus, it was confirmed that the efficiency of hybridization to a probe was increased by denaturation. The signal intensities were increased in the following order: 0.2 N NaOH (20°C) < heat treatment < 0.2 N NaOH (65°C). It was confirmed that alkali denaturation in 0.2 N NaOH at 65°C for 30 minutes can be preferably used for a polynucleotide. As observed in the absence of denaturation, the signal intensity observed for the product obtained by carrying out an ICAN reaction using Aminoallyl dUTP was higher than that observed for the product containing no Aminoallyl dUTP. The signal intensity was increased as the Aminoallyl dUTP content was increased. Based on these results, it was confirmed that the presence of the incorporated amino group increased the efficiency of immobilization of an ICAN-amplified nucleic acid onto a slide glass, and that the immobilization efficiency was increased as the content of the amino group to be incorporated was increased.

Example 6

Examination was carried out for an array having an immobilized practical double-stranded nucleic acid amplified by the ICAN method using the denaturation conditions found in Example 1. A chimeric oligonucleotide primer in a form of 5'-DNA-RNA-DNA-3' having amino group(s) incorporated into the DNA portion(s) on the 5'-terminal side and/or the 3'-terminal side was used for the examination.

(1) Preparation of template for ICAN reaction by PCR

A double-stranded cDNA was prepared using mouse brain polyA+ RNA (OriGene) and a cDNA synthesis kit (Takara Shuzo). A 340-bp amplification product was prepared by the PCR method using the double-stranded cDNA as a template and one of the following three pairs of primers for PCR: mouse ribosomal protein S18 F and R (SEQ ID NOS: 53 and 54); mouse glyceraldehyde-3-phosphate dehydrogenase F and R (SEQ ID NOS: 55 and 56); and mouse β-actin F and R (SEQ ID NOS: 57 and 58). A 422-bp amplification product was then prepared by the PCR method using the amplification product as a template and a pair of primers for PCR having nucleotide sequences of SEQ ID NOS: 59 and 60. The thus obtained amplified DNA fragment was used as a template for the ICAN method.

(2) Amplification of DNA fragment by ICAN method using PCR product as template

Chimeric oligonucleotide primers ICAN(2) and ICAN(6)6dN-M4 having nucleotide sequences of SEQ ID NOS: 61 and 62 were synthesized. The primers and the positions of incorporated amino groups are shown in Table 11.

Table 11

| No. | Primer pair |
|---|---|
| 1. | ICAN(6)6dN-M4 / ICAN(2) |
| 2. | $NH_2$-ICAN(6)6dN-M4 / ICAN(2) |
| 3. | ICAN(6)6dN-M4 25T-$NH_2$ / ICAN(2) |
| 4. | $NH_2$-ICAN(6)6dN-M4 25T-$NH_2$ / ICAN(2) |

In Table 11, for example, $NH_2$-ICAN(6)6dN-M4 25T-$NH_2$ / ICAN(2) represents a combination of a chimeric oligonucleotide primer ICAN(2) and a chimeric oligonucleotide primer ICAN(6)6dN having amino groups added to the 5' terminus and "T" at the 25th position.

10 µl of a mixture containing 100 pmol each of the primers in the pair, 100 ng of the PCR amplification product as described in (1) above as a template and propylenediamine at a final concentration of 0.01% was prepared. The mixture was incubated at 98°C for 2 minutes and then placed on ice. A reaction mixture (final volume of 50 µl) containing the mixture and, at final concentration, 28 mM potassium phosphate buffer (pH 7.8), 100 mM potassium acetate, 0.01% bovine serum albumin (BSA), 1% dimethyl sulfoxide, 4 mM magnesium acetate, 0.5 mM each of dNTPs, 8 U of BcaBEST DNA polymerase (Takara Shuzo) and 5 U of Pho RNase HII prepared as described in Referential Example 2 was prepared. The reaction mixture was mixed to homogeneity and incubated at 60°C for 60 minutes.

50 µl of isopropanol and 5 µl of a 3 M sodium acetate solution (pH5.2) were added to 50 µl of the reaction mixture. After allowing to stand at room temperature for 15 minutes, the mixture was centrifuged and a supernatant was removed. Subsequently, 200 µl of a 70% ethanol solution was added thereto, the mixture was centrifuged, a supernatant was removed, and the precipitate was air-dried. The thus obtained DNA was re-dissolved in water and the amount of the product was determined by measuring OD260/280.

(3) Preparation of DNA array using ICAN amplification product

The ICAN product was dissolved at a final concentration of 0.3 µg/µl in 40 mM carbonate buffer (pH 9.5) containing 0.1% sucrose monolaurate. For sample, three spots were spotted onto Mirus-E slide glass, which is a slide glass having an introduced activated carboxyl group, using Affymetrix 417 Arrayer. After spotting, the slide glass was washed in 0.2% SDS and in distilled water twice. After drying, the slide glass was treated as described in Example 2(2).

(4) Hybridization and detection

A fluorescence-labeled cDNA was prepared using a mouse total RNA and RNA Fluorescence Labeling Core Kit (M-MLV Version) (Takara Shuzo). Prehybridization, hybridization, washing and drying were carried out according to a protocol as described in instructions attached to IntelliGene using the fluorescence-labeled cDNA as a probe. Scanning was then carried out at the wavelength for Cy5 to obtain a fluorescence image. Signal intensities for the respective spots and the intensity for the surrounding background were quantified using ImaGene 4.0. As a result, it was confirmed that a utilization efficiency of a nucleic acid to be immobilized can be increased by incorporating an amino group and, resulting in immobilization of an amplified nucleic acid with high immobilization efficiency. In particular, it was confirmed that if a chimeric oligonucleotide primer in a form of DNA-RNA-DNA is to be used, a utilization efficiency of a nucleic acid to be immobilized is increased by incorporating amino groups in the DNA portions on the 5'-terminal side and the 3'-terminal side.

Industrial Applicability

An amplified nucleic acid for immobilization onto a solid phase can be provided in large quantities using a nucleic acid amplification reaction utilizing a chimeric oligonucleotide primer according to the present invention. An amplified nucleic acid that can be immobilized onto a solid phase utilizing a modified deoxyribonucleotide incorporated at the terminus of or in the amplified nucleic acid can be provided according to the method of the present invention. Furthermore, a material having an immobilized single-stranded nucleic acid containing a ribonucleotide or composed of deoxyribonucleotides can be provided according to the method of the present invention. A low-cost and highly sensitive material having an immobilized amplified nucleic acid can be provided according to the method of the present invention.

Sequence Listing Free Text

SEQ ID NO:2: PCR primer 1650Nde for cloning a gene encoding a polypeptide having a RNaseHII activity from Pyrococcus furiosus.
SEQ ID NO:3: PCR primer 1650Bam for cloning a gene encoding a polypeptide having a RNaseHII activity from Pyrococcus furiosus.
SEQ ID NO:6: Designed oligonucleotide.
SEQ ID NO:7: Designed chimeric oligonucleotide. "nucleotides 19 is ribonucleotide-other nucleotides are deoxyribonucleotides"
SEQ ID NO:8: Designed chimeric oligonucleotide. "nucleotides 19 to 20 are ribonucleotides-other nucleotides are deoxyribonucleotides"
SEQ ID NO:9: Designed chimeric oligonucleotide. "nucleotides 19 to 21 are ribonucleotides-other nucleotides are deoxyribonucleotides"
SEQ ID NO:10: Designed oligonucleotide which has a portion of succinate dehydrogenase-encoding sequence from Escherichia coli.
SEQ ID NO:11: Designed oligonucleotide probe-1 to detect a ICAN(6) sequence.
SEQ ID NO:12: Designed oligonucleotide probe to detect a M13-47 sequence.
SEQ ID NO:13: Designed cligonucleotide primer to amplify a portion of 131#6 sequence from Escherichia coli K-12 W3110.
SEQ ID NO:14: Designed oligonucleotide primer to amplify a portion of 131#6 sequence from Escherichia coli K-12 W3110.
SEQ ID NO:15: Designed oligonucleotide primer to amplify a portion of 214#1 sequence from Escherichia coli K-12 W3110.
SEQ ID NO:16: Designed oligonucleotide primer to amplify a portion of 214#1 sequence from Escherichia coli K-12 W3110."
SEQ ID NO:17: Designed oligonucleotide primer to amplify a portion of pUC19 plasmid DNA
SEQ ID NO:18: Designed oligonucleotide primer to amplify a portion of pUC19 plasmid DNA
SEQ ID NO:19: Designed chimeric oligonucleotide primer designated as ICAN(6). "nucleotides 19 to 20 are ribonucleotides-other nucleotides are deoxyribonucleotides"
SEQ ID NO:20: Designed chimeric oligonucleotide primer designated as ICAN(2). "nucleotides 19 to 20 are ribonucleotides-other nucleotides are deoxyribonucleotides"
SEQ ID NO:21: Designed oligonucleotide primer to amplify a portion of pUC19 plasmid DNA.
SEQ ID NO:22: Designed cligonucleotide primer to amplify a portion of pUC19 plasmid DNA.
SEQ ID NO:23: Designed oligonucleotide probe to detect a portion of pUC19 plasmid DNA.
SEQ ID NO:24: Designed oligonucleotide primer designated as ICAN(6)-M4.
SEQ ID NO:25: Designed oligonucleotide primer designated as ICAN(6)-RV.
SEQ ID NO:26: Designed oligonucleotide primer designated as ICANVFN3-M4.
SEQ ID NO:27: Designed oligonucleotide primer designated as ICANVFN3-RVM.
SEQ ID NO:28: Designed chimeric oligonucleotide primer designated as ICAN(6). "nucleotides 19 to 20 are ribonucleotides-other nucleotides are deoxyribonucleotides"
SEQ ID NO:29: Designed chimeric oligonucleotide primer designated as VFN3. "nucleotides 18 to 20 are ribonucleotides-other nucleotides are deoxyribonucleotides"
SEQ ID NO:30: Designed oligonucleotide primer to amplify a portion of ribosomal protein S18-encoding sequence from mouse.
SEQ ID NO:31: Designed oligonucleotide primer to amplify a portion of ribosomal protein S18-encoding sequence from mouse.
SEQ ID NO:32: Designed oligonucleotide primer to amplify a portion of transferrin receptor (TFR)-encoding sequence from mouse.
SEQ ID NO:33: Designed oligonucleotide primer to amplify a portion of transferrin receptor (TFR)-encoding sequence from mouse.
SEQ ID NO:34: Designed oligonucleotide primer to amplify a portion of stromal cell derived factor 4 (Sdf4)-encoding sequence from mouse.
SEQ ID NO:35: Designed oligonucleotide primer to amplify a portion of stromal cell derived factor 4 (Sdf4)-encoding sequence from mouse.

SEQ ID NO:36: Designed oligonucleotide primer to amplify a portion of cytoplasmic beta-actin encoding sequence from mouse.

SEQ ID NO:37: Designed oligonucleotide primer to amplify a portion of cytoplasmic beta-actin encoding sequence from mouse.

SEQ ID NO:38: Designed oligonucleotide primer to amplify a portion of ornithine decarboxylase-encoding sequence from mouse.

SEQ ID NO:39: Designed oligonucleotide primer to amplify a portion of ornithine decarboxylase-encoding sequence from mouse.

SEQ ID NO:40: Designed oligonucleotide primer to amplify a portion of hypoxanthine guanine phosphoribosyl transferase (HPRT)-encoding sequence from mouse.

SEQ ID NO:41: Designed oligonucleotide primer to amplify a portion of hypoxanthine guanine phosphoribosyl transferase (HPRT)-encoding sequence from mouse.

SEQ ID NO:42: Designed oligonucleotide primer to amplify a portion of tyrosine 3-monooxygenase encoding sequence from mouse.

SEQ ID NO:43: Designed oligonucleotide primer to amplify a portion of tyrosine 3-monooxygenase encoding sequence from mouse.

SEQ ID NO:44: Designed oligonucleotide primer designated as MCS-F.

SEQ ID NO:45: Designed oligonucleotide primer designated as MCS-R

SEQ ID NO:46: Designed oligonucleotide probe to detect a portion of extended products from MF2N3(24).

SEQ ID NO:47: Designed chimeric oligonucleotide primer designated as MF2N3(24). "nucleotides 22 to 24 are ribonucleoitdes-other nucleotides are deoxyribonucleotides"

SEQ ID NO:48: Designed chimeric oligonucleotide primer designated as MR1N3(24). "nucleotides 22 to 24 are ribonucleoitdes-other nucleotides are deoxyribonucleotides"

SEQ ID NO:49: Designed oligonucleotide primer designated as upper150 to amplify a portion of pUC19 plasmid DNA.

SEQ ID NO:50: Designed oligonucleotide primer designated as lower to amplify a portion of pUC19 plasmid DNA.

SEQ ID NO:53: PCR primer to amplify a portion of ribosomal protein S18 encoding sequence from mouse

SEQ ID NO:54: PCR primer to amplify a portion of ribosomal protein S18 encoding sequence from mouse

SEQ ID NO:55: PCR primer to amplify a portion of gluceraldehyde-3-phosphate dehydrogenase encoding sequence from mouse

SEQ ID NO:56: PCR primer to amplify a portion of gluceraldehyde-3-phosphate dehydrogenase encoding sequence from mouse.

SEQ ID NO:57: PCR primer to amplify a portion of beta-actin encoding sequence from mouse

SEQ ID NO:58: PCR primer to amplify a portion of beta-actin encoding sequence from mouse

SEQ ID NO:59: PCR primer to prepare a template for ICAN

SEQ ID NO:60: PCR primer to prepare a template for ICAN

SEQ ID NO:61: Designed chimeric oligonucleotide primer designated as ICAN(2). "nucleotides 19 to 20 are ribonucleoitdes-other nucleotides are deoxyribonucleotides"

SEQ ID NO:62: Designed chimeric oligonucleotide primer designated as ICAN(6) 6dN-M4. "nucleotides 19 to 20 are ribonucleoitdes-other nucleotides are deoxyribonucleotides"

## Claims

1. An amplified nucleic acid containing a deoxyribonucleotide modified for immobilization onto a solid phase, which is obtained by a nucleic acid amplification method comprising:

   (a) preparing a reaction mixture by mixing a nucleic acid as a template, deoxyribonucleotide triphosphates, a DNA polymerase having a strand displacement activity, at least one primer and an RNase H, wherein the primer is a chimeric oligonucleotide primer that is substantially complementary to the nucleotide sequence of the nucleic acid as the template and contains deoxyribonucleotides and a ribonucleotide, the ribonucleotide being positioned at the 3' terminus or on the 3'-terminal side of the primer, wherein a part of the deoxyribonucleotide triphosphates is a deoxyribonucleotide triphosphate modified for immobilization onto a solid phase and/or a part of the deoxyribonucleotides is a deoxyribonucleotide modified for immobilization onto a solid phase; and
   (b) incubating the reaction mixture for a sufficient time to generate a reaction product.

2. The amplified nucleic acid according to claim 1, wherein the deoxyribonucleotide modified for immobilization onto a solid phase is a modified deoxyribonucleotide having a functional group capable of covalently binding to a functional group on the solid phase.

3. The amplified nucleic acid according to claim 1, wherein the reaction mixture further contains a chimeric oligonucleotide primer having a sequence substantially homologous to the nucleotide sequence of the nucleic acid as the template.

4. The amplified nucleic acid according to claim 1, wherein a part of the deoxyribonucleotides is a deoxyribonucleotide modified for immobilization onto a solid phase.

5. The amplified nucleic acid according to claim 1, wherein a part of the deoxyribonucleotide triphosphates is a deoxyribonucleotide triphosphate modified for immobilization onto a solid phase.

6. The amplified nucleic acid according to claim 1, which contains a ribonucleotide.

7. The amplified nucleic acid according to claim 1, which does not contain a ribonucleotide.

8. The amplified nucleic acid according to claim 1, wherein the RNase H is selected from the group consisting of an RNase H from Escherichia coli, an RNase H from a bacterium of genus Thermotoga, an RNase H from a bacterium of genus Thermus, an RNase H from a bacterium of genus Pyrococcus and an RNase H from a bacterium of genus Bacillus.

9. The amplified nucleic acid according to claim 1, wherein the RNase H is type I RNase H from Escherichia coli

10. The amplified nucleic acid according to claim 1, wherein the RNase H is type II RNase H from Pyrococcus furiosus or Pyrococcus horikoshii.

11. A material having an immobilized amplified nucleic acid in which the amplified nucleic acid defined by claim 1 is immobilized and arrayed in a predefined region on a solid phase.

12. The material according to claim 11, wherein the amplified nucleic acid is immobilized via a covalent bond.

13. A material having an immobilized amplified nucleic acid, which is obtained by a method comprising:

   (a) preparing a reaction mixture by mixing a nucleic acid as a template, a deoxyribonucleotide triphosphate, a DNA polymerase having a strand displacement activity, two primers and an RNase H, wherein one primer is a chimeric oligonucleotide primer that is substantially complementary to the nucleotide sequence of the nucleic acid as the template and contains deoxyribonucleotides and a ribonucleotide, the ribonucleotide being positioned at the 3' terminus or on the 3'-terminal side of the primer, a part of the deoxyribonucleotides being a deoxyribonucleotide modified for immobilization onto a solid phase, and another primer is a chimeric oligonucleotide primer that is substantially homologous to the nucleotide sequence of the nucleic acid as the template and does not contain a deoxyribonucleotide modified for immobilization onto a solid phase;

(b) incubating the reaction mixture for a sufficient time to generate a reaction product;
(c) immobilizing and arraying the amplified nucleic acid obtained in step (b) in a predefined region on a solid phase; and
(d) releasing only the strand extended from the chimeric oligonucleotide primer that does not contain a deoxyribonucleotide modified for immobilization onto a solid phase.

**14.** The material according to claim 13, wherein the amplified nucleic acid is immobilized via a covalent bond in step (c).

**15.** A chimeric oligonucleotide primer represented by general formula below:

General formula: 5'-dNa-Nb-dNc-3'

(a: an integer of 11 or more; b: an integer of 1 or more; c: 0 or an integer of 1 or more; dN: deoxyribonucleotide and/or deoxyribonucleotide analog, wherein at least one of dNs is a deoxyribonucleotide modified for immobilization onto a solid phase; N: unmodified ribonucleotide and/or modified ribonucleotide, wherein some of dNs in dNa may be replaced by Ns).

**16.** The chimeric oligonucleotide primer according to claim 15, wherein c is 0.

**17.** The chimeric oligonucleotide primer according to claim 15, wherein the deoxyribonucleotide analog is deoxyriboinosine nucleotide and the modified ribonucleotide is ($\alpha$-S) ribonucleotide.

**18.** A method for producing an amplified nucleic acid containing a deoxyribonucleotide modified for immobilization onto a solid phase, the method comprising:

(a) preparing a reaction mixture by mixing a nucleic acid as a template, deoxyribonucleotide triphosphates, a DNA polymerase having a strand displacement activity, at least one primer and an RNase H, wherein the primer is a chimeric oligonucleotide primer that is substantially complementary to the nucleotide sequence of the nucleic acid as the template and contains deoxyribonucleotides and a ribonucleotide, the ribonucleotide being positioned at the 3' terminus or on the 3'-terminal side of the primer, wherein a part of the deoxyribonucleotide triphosphates is a deoxyribonucleotide triphosphate modified for immobilization onto a solid phase and/or a part of the deoxyribonucleotides is a deoxyribonucleotide modified for immobilization onto a solid phase; and
(b) incubating the reaction mixture for a sufficient time to generate a reaction product.

**19.** A method for producing a material having an immobilized amplified nucleic acid, the method comprising:

(a) preparing a reaction mixture by mixing a nucleic acid as a template, deoxyribonucleotide triphosphates, a DNA polymerase having a strand displacement activity, at least one primer and an RNase H, wherein the primer is a chimeric oligonucleotide primer that is substantially complementary to the nucleotide sequence of the nucleic acid as the template and contains deoxyribonucleotides and a ribonucleotide, the ribonucleotide being positioned at the 3' terminus or on the 3'-terminal side of the primer, wherein a part of the deoxyribonucleotide triphosphates is a deoxyribonucleotide triphosphate modified for immobilization onto a solid phase and/or a part of the deoxyribonucleotides is a deoxyribonucleotide modified for immobilization onto a solid phase;
(b) incubating the reaction mixture for a sufficient time to generate a reaction product; and
(c) immobilizing and arraying the amplified nucleic acid obtained in step (b) in a predefined region on a solid phase.

**20.** A method for producing a material having an immobilized amplified nucleic acid, the method comprising:

(a) preparing a reaction mixture by mixing a nucleic acid as a template, a deoxyribonucleotide triphosphate, a DNA polymerase having a strand displacement activity, two primers and an RNase H, wherein one primer is a chimeric oligonucleotide primer that is substantially complementary to the nucleotide sequence of the nucleic acid as the template and contains deoxyribonucleotides and a ribonucleotide, the ribonucleotide being positioned at the 3' terminus or on the 3'-terminal side of the primer, a part of the deoxyribonucleotides being a deoxyribonucleotide modified for immobilization onto a solid phase, and another primer is a chimeric oligonucleotide primer that is substantially homologous to the nucleotide sequence of the nucleic acid as the template and does not contain a deoxyribonucleotide modified for immobilization onto a solid phase;
(b) incubating the reaction mixture for a sufficient time to generate a reaction product;
(c) immobilizing and arraying the amplified nucleic acid obtained in step (b) in a predefined region on a solid phase; and
(d) releasing only the strand extended from the chimeric oligonucleotide primer that does not contain a deoxyribonucleotide modified for immobilization onto a solid phase.

# INTERNATIONAL SEARCH REPORT

International application No.
PCT/JP02/00941

**A. CLASSIFICATION OF SUBJECT MATTER**
Int.Cl[7] C12N15/11, C12Q1/68

According to International Patent Classification (IPC) or to both national classification and IPC

**B. FIELDS SEARCHED**

Minimum documentation searched (classification system followed by classification symbols)
Int.Cl[7] C12N15/11, C12Q1/68, C07H21/00-21/04

Documentation searched other than minimum documentation to the extent that such documents are included in the fields searched

Electronic data base consulted during the international search (name of data base and, where practicable, search terms used)
JICST FILE(JOIS), WPI(DIALOG), BIOSIS(DIALOG), MEDLINE(STN), PCI(DIALOG)

**C. DOCUMENTS CONSIDERED TO BE RELEVANT**

| Category* | Citation of document, with indication, where appropriate, of the relevant passages | Relevant to claim No. |
|---|---|---|
| Y | WO 93/09250 A1 (Adelaide Children's Hospital), 13 May, 1993 (13.05.93), & AU 9228723 A & BR 9206705 A & EP 672173 A1 & US 6017738 A Description, pages 6 to 10; Figs. 5a, 5b, 6 | 1-20 |
| Y | WO 00/56877 A1 (Takara Shuzo Co., Ltd.), 28 September, 2000 (28.09.00), & AU 200029442 A & EP 1167524 A1 | 1-20 |
| Y | Ikunoshin KATO, ICAN-ho ha PCR o Koemasuka?, Kagaku, 01 December, 2000 (01.12.00), Vol.55, No.12, pages 20 to 21 | 1-20 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 May, 2002 (08.05.02) | 21 May, 2002 (21.05.02) |
| Name and mailing address of the ISA/ Japanese Patent Office | Authorized officer |
| Facsimile No. | Telephone No. |

Form PCT/ISA/210 (second sheet) (July 1998)

## INTERNATIONAL SEARCH REPORT

International application No.
PCT/JP02/00941

C (Continuation). DOCUMENTS CONSIDERED TO BE RELEVANT

| Category* | Citation of document, with indication, where appropriate, of the relevant passages | Relevant to claim No. |
|---|---|---|
| Y | Ryotaro SAKATA, Takara Shuzo, Sachikanho o Mochiita DNA to Onzofuku Gijutsu o Kaihatsu, DNA Tairyo Seisan ga Kano ni, Nikkei Biotech, 09 October, 2000 (09.10.00), No.457, pages 4 to 5 | 1-20 |
| A | EP 374665 A2 (Molecular Diagnostics, Inc.), 27 June, 1990 (27.06.90), & PT 92564 A & AU 8947144 A & CA 2004326 A & DK 8906627 A & JP 3-43099 A & AU 9337162 A | 1-20 |
| A | EP 297379 A2 (Molecular Diagnostics, Inc.), 04 January, 1989 (04.01.89), & CA 1317535 B & DK 3873842 B & JP 1-98499 A | 1-20 |
| A | WO 94/29484 A1 (Gamera Bioscience Corp.), 22 December, 1994 (22.12.94), & AU 9471067 A & US 5545540 A & EP 702728 A1 & JP 8-511425 A & DE 69409646 B & ES 2114692 B & CA 2164706 B | 1-20 |

Form PCT/ISA/210 (continuation of second sheet) (July 1998)

SEQUENCE LISTING

<110> Takara Bio Inc.

<120> Amplified nucleic acids and immobilized products thereof

<130> 38-61

<140> EP 02 711 315.8

<141> 2002-02-06

<150> JP 2001-030153

<151> 2001-02-06

<160> 62

<210> 1

<211> 663

<212> DNA

<213> Pyrococcus horikoshii

<400> 1

atgaaggttg ctggagttga tgaagcgggg agggggccgg taattggccc gttagtaatt 60

ggagtagccg ttatagatga gaaaaatatt gagaggttac gtgacattgg ggttaaagac 120

tccaaacaat taactcctgg gcaacgtgaa aaactattta gcaaattaat agatatccta 180

gacgattatt atgttcttct cgttaccccc aaggaaatag atgagaggca tcattctatg 240

aatgaactag aagctgagaa attcgttgta gccttgaatt ctttaaggat caagccgcag 300

aagatatatg tggactctgc cgatgtagat cctaagaggt ttgctagtct aataaaggct 360

gggttgaaat atgaagccac ggttatcgcc gagcataaag ccgatgcaaa gtatgagata 420

gtatcggcag catcaataat tgcaaaggtc actagggata gagagataga gaagctaaag 480

caaaagtatg gggaatttgg ttctggctat ccgagtgatc cgagaactaa ggagtggctt 540

gaagaatatt acaaacaata tggtgacttt cctccaatag ttaggagaac ttgggaaacc 600

gctaggaaga tagaggaaag gtttagaaaa aatcagctaa cgcttgataa attccttaag 660

tga 663

<210> 2

<211> 33

<212> DNA

<213> Artificial Sequence

<220>

<223> PCR primer 1650Nde for cloning a gene encoding a polypeptide having a RNaseHII activity from Pyrococcus furiosus

<400> 2

caggaggaga gacatatgaa aataggggga att 33

<210> 3

<211> 33

<212> DNA

<213> Artificial Sequence

<220>

<223> PCR primer 1650Bam for cloning a gene encoding a polypeptide having a RNaseHII activity from Pyrococcus furiosus

<400> 3

gaaggttgtg gatccacttt ctaaggtttc tta 33

<210> 4

<211> 672

<212> DNA

<213> Pyrococcus furiosus

<400> 4

ATGAAAATAG GGGGAATTGA CGAAGCAGGA AGAGGACCAG CGATAGGGCC ATTAGTAGTA 60

GCTACTGTCG TCGTTGATGA GAAAAACATT GAGAAGCTCA GAAACATTGG AGTAAAAGAC 120

TCCAAACAAC TAACACCCCA TGAAAGGAAG AATTTATTTT CCCAGATAAC CTCAATAGCG 180

GATGATTACA AAATAGTGAT AGTATCCCCA GAAGAAATCG ACAATAGATC AGGAACAATG 240

AACGAGTTAG AGGTAGAGAA GTTTGCTCTC GCCTTAAATT CGCTTCAGAT AAAACCAGCT 300

CTTATATACG CTGATGCAGC GGATGTAGAT GCCAATAGAT TTGCAAGCTT GATAGAGAGA 360

AGACTCAATT ATAAGGCGAA GATTATTGCC GAACACAAGG CCGATGCAAA GTATCCAGTA 420

GTTTCAGCAG CTTCAATACT TGCAAAGGTT GTTAGGGATG AGGAAATTGA AAAATTAAAA 480

AAGCAATATG GAGACTTTGG CTCTGGGTAT CCAAGTGATC CAAAAACCAA GAAATGGCTT 540

GAAGAGTACT ACAAAAAACA CAACTCTTTC CCTCCAATAG TCAGACGAAC CTGGGAAACT 600

GTAAGAAAAA TAGAGGAAAG CATTAAAGCC AAAAAATCCC AGCTAACGCT TGATAAATTC 660

TTTAAGAAAC CT 672

<210> 5

<211> 224

<212> PRT

<213> Pyrococcus furiosus

<400> 5

Met Lys Ile Gly Gly Ile Asp Glu Ala Gly Arg Gly Pro Ala Ile
1 5 10 15

Gly Pro Leu Val Val Ala Thr Val Val Val Asp Glu Lys Asn Ile

20 25 30

Glu Lys Leu Arg Asn Ile Gly Val Lys Asp Ser Lys Gln Leu Thr

35 40 45

Pro His Glu Arg Lys Asn Leu Phe Ser Gln Ile Thr Ser Ile Ala

50 55 60

Asp Asp Tyr Lys Ile Val Ile Val Ser Pro Glu Glu Ile Asp Asn

65 70 75

Arg Ser Gly Thr Met Asn Glu Leu Glu Val Glu Lys Phe Ala Leu

80 85 90

Ala Leu Asn Ser Leu Gln Ile Lys Pro Ala Leu Ile Tyr Ala Asp

95 100 105

Ala Ala Asp Val Asp Ala Asn Arg Phe Ala Ser Leu Ile Glu Arg

110 115 120

Arg Leu Asn Tyr Lys Ala Lys Ile Ile Ala Glu His Lys Ala Asp

125 130 135

Ala Lys Tyr Pro Val Val Ser Ala Ala Ser Ile Leu Ala Lys Val

140 145 150

Val Arg Asp Glu Glu Ile Glu Lys Leu Lys Lys Gln Tyr Gly Asp

155 160 165

Phe Gly Ser Gly Tyr Pro Ser Asp Pro Lys Thr Lys Lys Trp Leu

170 175 180

Glu Glu Tyr Tyr Lys Lys His Asn Ser Phe Pro Pro Ile Val Arg

185 190 195

Arg Thr Trp Glu Thr Val Arg Lys Ile Glu Glu Ser Ile Lys Ala

200 205 210

Lys Lys Ser Gln Leu Thr Leu Asp Lys Phe Phe Lys Lys Pro

215 220

<210> 6

<211> 42

<212> DNA

<213> Artificial Sequence

<220>

<223> Designed oligonucleotide.

<400> 6

acatcacagt agtcgttcgt cgtgactggg aaaaccctgg cg 42

<210> 7

<211> 43

<212> DNA

<213> Artificial Sequence

<220>

<223> Designed chimeric oligonucleotide. "nucleotides 19 is ribonucleotide-other nucleotides are deoxyribonucleotides"

<400> 7

acatcacagt agtcgttcag tcgtgactgg gaaaaccctg gcg 43

<210> 8

<211> 44

<212> DNA

<213> Artificial Sequence

<220>

<223> Designed chimeric oligonucleotide. "nucleotides 19 to 20 are ribonucleotides-other nucleotides are deoxyribonucleotides"

<400> 8

acatcacagt agtcgttcac gtcgtgactg ggaaaaccct ggcg 44

<210> 9

<211> 45

<212> DNA

<213> Artificial Sequence

<220>

<223> Designed chimeric oligonucleotide. "nucleotides 19 to 21 are ribonucleotides-other nucleotides are deoxyribonucleotides"

<400> 9

acatcacagt agtcgttcac agtcgtgact gggaaaaccc tggcg 45

<210> 10

<211> 40

<212> DNA

<213> Artificial Sequence

<220>

<223> Designed oligonucleotide which has a portion of succinate dehydrogenase-encoding sequence from Escherichia coli.

<400> 10

aggacgcaat ggcgtacatg atttcatcct cgttcgcgct 40

<210> 11

<211> 20

<212> DNA

<213> Artificial Sequence

<220>

<223> Designed oligonucleotide probe-1 to detect a ICAN(6) sequence.

<400> 11

gtgaacgact actgtgatgt 20

<210> 12

<211> 24

<212> DNA

<213> Artificial Sequence

<220>

<223> Designed oligonucleotide probe to detect a M13-47 sequence.

<400> 12

cgccagggtt ttcccagtca cgac 24

<210> 13

<211> 24

<212> DNA

<213> Artificial Sequence

<220>

<223> Designed oligonucleotide primer to amplify a portion of 131#6 sequence from Escherichia coli K-12 W3110.

<400> 13

gtggaggcaa acccgtccgt gtgt 24

<210> 14

<211> 24

<212> DNA

<213> Artificial Sequence

<220>

<223> Designed oligonucleotide primer to amplify a portion of 131#6 sequence from Escherichia coli K-12 W3110.

<400> 14

ttacccggta acagatatat cgcc 24

<210> 15

<211> 24

<212> DNA

<213> Artificial Sequence

<220>

<223> Designed oligonucleotide primer to amplify a portion of 214#1 sequence from Escherichia coli K-12 W3110.

<400> 15

atggccaaag aagacaatat tgaa 24

<210> 16

<211> 24

<212> DNA

<213> Artificial Sequence

<220>

<223> Designed oligonucleotide primer to amplify a portion of 214#1 sequence from Escherichia coli K-12 W3110."

<400> 16

tcagcgacta cggaagacaa tgcg 24

<210> 17

<211> 52

<212> DNA

<213> Artificial Sequence

<220>

<223> Designed oligonucleotide primer to amplify a portion of pUC19 plasmid DNA

<400> 17

ccacagcgcc atttcacatc acagtagtcg ttcacgtttt cccagtcacg ac 52

<210> 18

<211> 52

<212> DNA

<213> Artificial Sequence

<220>

<223> Designed oligonucleotide primer to amplify a portion of pUC19 plasmid DNA

<400> 18

ccgtacaact atttcactga ctagctgtag cacaccagga aacagctatg ac 52

<210> 19

<211> 20

<212> DNA

<213> Artificial Sequence

<220>

<223> Designed chimeric oligonucleotide primer designated as ICAN(6). "nucleotides 19 to 20 are ribonucleotides-other nucleotides are deoxyribonucleotides"

<400> 19

acatcacagt agtcgttcac 20

<210> 20

<211> 20

<212> DNA

<213> Artificial Sequence

<220>

<223> Designed chimeric oligonucleotide primer designated as ICAN(2). "nucleotides 19 to 20 are ribonucleotides-other nucleotides are deoxyribonucleotides"

<400> 20

actgactagc tgtagcacac 20

<210> 21

<211> 17

<212> DNA

<213> Artificial Sequence

<220>

<223> Designed oligonucleotide primer to amplify a portion of pUC19 plasmid DNA.

<400> 21

gttttcccag tcacgac 17

<210> 22

<211> 17

<212> DNA

<213> Artificial Sequence

<220>

<223> Designed oligonucleotide primer to amplify a portion of pUC19 plasmid DNA.

<400> 22

caggaaacag ctatgac 17

<210> 23

<211> 21

<212> DNA

<213> Artificial Sequence

<220>

<223> Designed oligonucleotide probe to detect a portion of pUC19 plasmid DNA.

<400> 23

atgaccatga ttacgccaag c 21

<210> 24

<211> 74

<212> DNA

<213> Artificial Sequence

<220>

<223> Designed oligonucleotide primer designated as ICAN(6)-M4.

<400> 24

ctgatgagtt cgtgtccgta caactatttc acatcacagt agtcgttcac cgccagggtt 60

ttcccagtca cgac 74

<210> 25

<211> 55

<212> DNA

<213> Artificial Sequence

<220>

<223> Designed oligonucleotide primer designated as ICAN(6)-RV.

<400> 25

ccgtacaact atttcacatc acagtagtcg ttcacacaca ggaaacagct atgac 55

<210> 26

<211> 70

<212> DNA

<213> Artificial Sequence

<220>

<223> Designed oligonucleotide primer designated as ICANVFN3-M4.

<400> 26

ctgatgagtt cgtgtccgta caactatttc tactgggttt ttcttcggta agggttttcc 60

cagtcacgac 70

<210> 27

<211> 74

<212> DNA

<213> Artificial Sequence

<220>

<223> Designed oligonucleotide primer designated as ICANVFN3-RVM.

<400> 27

ggttatcgaa atcagccaca gcgccatttc tactgggttt ttcttcggta gagcggataa 60

caatttcaca cagg 74

<210> 28

<211> 20

<212> DNA

<213> Artificial Sequence

<220>

<223> Designed chimeric oligonucleotide primer designated as ICAN(6). "nucleotides 19 to 20 are ribonucleotides-other nucleotides are deoxyribonucleotides"

<400> 28

acatcacagt agtcgttcac 20

<210> 29

<211> 20

<212> DNA

<213> Artificial Sequence

<220>

<223> Designed chimeric oligonucleotide primer designated as VFN3. "nucleotides 18 to 20 are ribonucleotides-other nucleotides are deoxyribonucleotides"

<400> 29

tactgggttt ttcttcggua 20

<210> 30

<211> 23

<212> DNA

<213> Artificial Sequence

<220>

<223> Designed oligonucleotide primer to amplify a portion of ribosomal protein S18-encoding sequence from mouse.

<400> 30

gtctctagtg atccctgaga agt 23

<210> 31

<211> 23

<212> DNA

<213> Artificial Sequence

<220>

<223> Designed oligonucleotide primer to amplify a portion of ribosomal protein S18-encoding sequence from mouse.

<400> 31

tggatacacc cacagttcgg ccc 23

<210> 32

<211> 23

<212> DNA

<213> Artificial Sequence

<220>

<223> Designed oligonucleotide primer to amplify a portion of transferrin receptor (TFR)-encoding sequence from mouse.

<400> 32

ccgcgctccg acaagtagat gga 23

<210> 33

<211> 23

<212> DNA

<213> Artificial Sequence

<220>

<223> Designed oligonucleotide primer to amplify a portion of transferrin receptor (TFR)-encoding sequence from mouse.

<400> 33

ccaaagagtg caaggtctgc ctc 23

<210> 34

<211> 23

<212> DNA

<213> Artificial Sequence

<220>

<223> Designed oligonucleotide primer to amplify a portion of stromal cell derived factor 4 (Sdf4)-encoding sequence from mouse.

<400> 34

tctgatggat gcaaccgcta gac 23

<210> 35

<211> 23

<212> DNA

<213> Artificial Sequence

<220>

<223> Designed oligonucleotide primer to amplify a portion of stromal cell derived factor 4 (Sdf4)-encoding sequence from mouse.

<400> 35

gaactcttca tgcacgttgc ggg 23

<210> 36

<211> 23

<212> DNA

<213> Artificial Sequence

<220>

<223> Designed oligonucleotide primer to amplify a portion of cytoplasmic beta-actin encoding sequence from mouse.

<400> 36

tgatggtggg aatgggtcag aag 23

<210> 37

<211> 23

<212> DNA

<213> Artificial Sequence

<220>

<223> Designed oligonucleotide primer to amplify a portion of cytoplasmic beta-actin encoding sequence from mouse.

<400> 37
agaagcactt gcggtgcacg atg 23

<210> 38
<211> 23
<212> DNA
<213> Artificial Sequence

<220>
<223> Designed oligonucleotide primer to amplify a portion of ornithine decarboxylase-encoding sequence from mouse.

<400> 38
gatgaaagtc gccagagcac atc 23

<210> 39
<211> 23
<212> DNA
<213> Artificial Sequence

<220>
<223> Designed oligonucleotide primer to amplify a portion of ornithine decarboxylase-encoding sequence from mouse.

<400> 39
ttgatcctag cagaagcaca ggc 23

<210> 40

<211> 23

<212> DNA

<213> Artificial Sequence

<220>

<223> Designed oligonucleotide primer to amplify a portion of hypoxanthine guanine phosphoribosyl transferase (HPRT)-encoding sequence from mouse.

<400> 40

ggacaggact gaaagacttg ctc 23

<210> 41

<211> 23

<212> DNA

<213> Artificial Sequence

<220>

<223> Designed oligonucleotide primer to amplify a portion of hypoxanthine guanine phosphoribosyl transferase (HPRT)-encoding sequence from mouse.

<400> 41

gtctggcctg tatccaacac ttc 23

<210> 42

<211> 23

<212> DNA

<213> Artificial Sequence

<220>

<223> Designed oligonucleotide primer to amplify a portion of tyrosine 3-monooxygenase encoding sequence from mouse.

<400> 42

atgagctggt gcagaaggcc aag 23

<210> 43

<211> 23

<212> DNA

<213> Artificial Sequence

<220>

<223> Designed oligonucleotide primer to amplify a portion of tyrosine 3-monooxygenase encoding sequence from mouse.

<400> 43

ttcccctcct tctcctgctt ctg 23

<210> 44

<211> 21

<212> DNA

<213> Artificial Sequence

<220>

<223> Designed oligonucleotide primer designated as MCS-F.

<400> 44

ccattcaggc tgcgcaatgt t 21

<210> 45

<211> 22

<212> DNA

<213> Artificial Sequence

<220>

<223> Designed oligonucleotide primer designated as MCS-R

<400> 45

tggcacgaca ggtttcccga ct 22

<210> 46

<211> 21

<212> DNA

<213> Artificial Sequence

<220>

<223> Designed oligonucleotide probe to detect a portion of extended products from MF2N3(24).

<400> 46

atgaccatga ttacgccaag c 21

<210> 47

<211> 24

<212> DNA

<213> Artificial Sequence

<220>

<223> Designed chimeric oligonucleotide primer designated as MF2N3(24). "nucleotides 22 to 24 are ribonucleoitdes-other nucleotides are deoxyribonucleotides"

<400> 47

gctgcaaggc gattaagttg ggua 24

<210> 48

<211> 24

<212> DNA

<213> Artificial Sequence

<220>

<223> Designed chimeric oligonucleotide primer designated as MR1N3(24). "nucleotides 22 to 24 are ribonucleoitdes-other nucleotides are deoxyribonucleotides"

<400> 48

ctttatgctt ccggctcgta tguu 24

<210> 49

<211> 25

<212> DNA

<213> Artificial Sequence

<220>

<223> Designed oligonucleotide primer designated as upper150 to amplify a portion of pUC19 plasmid DNA.

<400> 49

ggtgtcacgc tcgtcgtttg gtatg 25

<210> 50

<211> 25

<212> DNA

<213> Artificial Sequence

<220>

<223> Designed oligonucleotide primer designated as lower to amplify a portion of pUC19 plasmid DNA.

<400> 50

ggaaacactg cggccaactt acttc 25

<210> 51

<211> 673

<212> DNA

<213> Pyrococcus horikoshii

<400> 51

atgaaggttg ctggagttga tgaagcgggg agggggccgg taattggccc gttagtaatt 60
ggagtagccg ttatagatga gaaaaatatt gagaggttac gtgacattgg ggttaaagac 120
tccaaacaat taactcctgg gcaacgtgaa aaactattta gcaaattaat agatatccta 180
gacgattatt atgttcttct cgttaccccc aaggaaatag atgagaggca tcattctatg 240

aatgaactag aagctgagaa attcgttgta gccttgaatt ctttaaggat caagccgcag 300

aagatatatg tggactctgc cgatgtagat cctaagaggt ttgctagtct aataaaggct 360

gggttgaaat atgaagccac ggttatcgcc gagcataaag ccgatgcaaa gtatgagata 420

gtatcggcag catcaataat tgcaaaggtc actagggata gagagataga gaagctaaag 480

caaaagtatg gggaatttgg ttctggctat ccgagtgatc cgagaactaa ggagtggctt 540

gaagaatatt acaaacaata tggtgacttt cctccaatag ttaggagaac ttgggaaacc 600

gctaggaaga tagaggaaag gtttagaaaa aatcagctaa cgcttgataa attccttaag 660

tgatcttgga tcc 673

<210> 52

<211> 220

<212> PRT

<213> Pyrococcus horikoshii

<400> 52

Met Lys Val Ala Gly Val Asp Glu Ala Gly Arg Gly Pro Val Ile
1 5 10 15

Gly Pro Leu Val Ile Gly Val Ala Val Ile Asp Glu Lys Asn Ile
20 25 30

Glu Arg Leu Arg Asp Ile Gly Val Lys Asp Ser Lys Gln Leu Thr
35 40 45

Pro Gly Gln Arg Glu Lys Leu Phe Ser Lys Leu Ile Asp Ile Leu
50 55 60

Asp Asp Tyr Tyr Val Leu Leu Val Thr Pro Lys Glu Ile Asp Glu
65 70 75

Arg His His Ser Met Asn Glu Leu Glu Ala Glu Lys Phe Val Val
80 85 90

Ala Leu Asn Ser Leu Arg Ile Lys Pro Gln Lys Ile Tyr Val Asp
95 100 105

Ser Ala Asp Val Asp Pro Lys Arg Phe Ala Ser Leu Ile Lys Ala

110 115 120

Gly Leu Lys Tyr Glu Ala Thr Val Ile Ala Glu His Lys Ala Asp

125 130 135

Ala Lys Tyr Glu Ile Val Ser Ala Ala Ser Ile Ile Ala Lys Val

140 145 150

Thr Arg Asp Arg Glu Ile Glu Lys Leu Lys Gln Lys Tyr Gly Glu

155 160 165

Phe Gly Ser Gly Tyr Pro Ser Asp Pro Arg Thr Lys Glu Trp Leu

170 175 180

Glu Glu Tyr Tyr Lys Gln Tyr Gly Asp Phe Pro Pro Ile Val Arg

185 190 195

Arg Thr Trp Glu Thr Ala Arg Lys Ile Glu Glu Arg Phe Arg Lys

200 205 210

Asn Gln Leu Thr Leu Asp Lys Phe Leu Lys

215 220

<210> 53

<211> 40

<212> DNA

<213> Artificial Sequence

<220>

<223> PCR primer to amplify a portion of ribosomal protein S18 encoding sequence from mouse

<400> 53

agggttttcc cagtcacgac caacatcgat gggcggcgga 40

<210> 54

<211> 40

<212> DNA

<213> Artificial Sequence

<220>

<223> PCR primer to amplify a portion of ribosomal protein S18 encoding sequence from mouse

<400> 54

acacaggaaa cagctatgac cgaattttct tcagcctctc 40

<210> 55

<211> 40

<212> DNA

<213> Artificial Sequence

<220>

<223> PCR primer to amplify a portion of gluceraldehyde-3-phosphate dehydrogenase encoding sequence from mouse

<400> 55

agggttttcc cagtcacgac gtgggcaagg tcatcccaga 40

<210> 56

<211> 40

<212> DNA

<213> Artificial Sequence

<220>

<223> PCR primer to amplify a portion of gluceraldehyde-3-phosphate dehydrogenase encoding sequence from mouse.

<400> 56

acacaggaaa cagctatgac attgtcatac caggaaatga 40

<210> 57

<211> 40

<212> DNA

<213> Artificial Sequence

<220>

<223> PCR primer to amplify a portion of beta-actin encoding sequence from mouse

<400> 57

agggttttcc cagtcacgac gccatcctgc gtctggactt 40

<210> 58

<211> 40

<212> DNA

<213> Artificial Sequence

<220>

<223> PCR primer to amplify a portion of beta-actin encoding sequence from mouse

<400> 58

acacaggaaa cagctatgac gccacaggat tccataccca 40

<210> 59

<211> 61

<212> DNA

<213> Artificial Sequence

<220>

<223> PCR primer to prepare a template for ICAN

<400> 59

cgactatagg ccaattcggc cacatcacag tagtcgttca cagggttttc ccagtcacga 60

c 61

<210> 60

<211> 61

<212> DNA

<213> Artificial Sequence

<220>

<223> PCR primer to prepare a template for ICAN

<400> 60

gacctgcagg ccattatggc cactgactag ctgtagcaca cacacaggaa acagctatga 60

c 61

<210> 61

<211> 20

<212> DNA

<213> Artificial Sequence

<220>

<223> Designed chimeric oligonucleotide primer designated as ICAN(2). "nucleotides 19 to 20 are ribonucleoitdes-other nucleotides are deoxyribonucleotides"

<400> 61

actgactagc tgtagcacac 20

<210> 62

<211> 26

<212> DNA

<213> Artificial Sequence

<220>

<223> Designed chimeric oligonucleotide primer designated as ICAN(6) 6dN-M4. "nucleotides 19 to 20 are ribonucleoitdes-other nucleotides are deoxyribonucleotides"

<400> 62

acatcacagt agtcgttcac agggtt 26